# EUROPEAN PATENT APPLICATION

(11) **EP 1 361 435 A1**
(43) Date of publication of application: **12.11.2003**
(21) Application number: 02800234.3
(22) Date of filing: 17.09.2002
(51) Int. Cl.: G01N 33/543

(54) **SPECIFIC BINDING ANALYZER AND METHOD OF ANALYZING SPECIFIC BINDING**

(30) Priority: 28.09.2001 JP 2001302340; 17.12.2001 JP 2001383358
(71) Applicant: Matsushita Electric Industrial Co., Ltd., Kadoma-shi, Osaka 571-8501 (JP)
(72) Inventor: KENJYOU, Noriko, Hirakata-shi, Osaka 573-0036 (JP); KAMEI, Akihito, Yawata-shi, Kyoto 614-8295 (JP); KAWAMURA, Tatsurou, Kyotanabe-shi, Kyoto 610-0351 (JP)
(74) Representative: Leson, Thomas Johannes Alois, Dipl.-Ing.
(86) International application number: JP0209527
(87) International publication number: WO03029822

(57) **Abstract**

In order to perform a qualitative or quantitative analysis of an analyte in a sample in a simple, rapid and accurate manner where the sample can be automatically diluted without the need for an advanced device or operation, under little influence attributed to the analyte concentration in the sample, a strip is used which comprises at least two or more units arranged therein, each unit comprising: a space forming zone 2; a retention zone 5 in which a labeled first specific binding substance is retained; and a detection zone 6 in which a second specific binding substance is immobilized, so that the analyte in the sample is qualitatively or quantitatively analyzed based on a signal intensity obtained attributed to a specific binding reaction in the detection zone 6.

## Description

### Technical Field

The present invention relates to a specific binding analysis device and a specific binding analysis method for qualitatively or quantitatively analyzing an analyte in a sample in a simple and rapid manner.

### Background Art

With the recent expansion of medical care in households and communities as well as increase of clinical examinations with high urgency, there is an increasing demand for development of a specific binding analysis method which can be performed even by persons other than experts of the clinical examination, in a rapid, simple and reliable manner.

Many methods are known as the specific binding analysis methods, which include immunoassay applying an antigen-antibody reaction, receptor assay employing a receptor and nucleic acid probe assay employing hybridization of complementary nucleic acid sequences, and these methods have been widely used in the clinical examinations and many other fields because of the high specificity thereof.

As a more specific example mentioned can be chromatography which comprises an operation of making a sample suspected of containing an analyte infiltrate and develop in a chromatograph region, which comprises a porous carrier or a fine particle-packed carrier and where a specific binding substance is insolubilized, together with, or separated from, a labeled specific binding substance. This analysis method is a type of immunoassay.

Chromatography is advantageous in terms of measurement sensitivity and measurement time since the carrier in the chromatography region has a large surface area where a great amount of the specific binding substance can be insolubilized, and a collision between reacting molecules, which may cause a specific binding reaction, occurs with a higher frequency as compared with a reaction in a liquid phase.

However, the conventional specific binding analysis method has a problem called a prozone phenomenon. This is a phenomenon in which an analyte concentration cannot be unambiguously determined with respect to a signal intensity attributed to a specific binding reaction.

In a specific binding analysis method, an analyte is qualitatively or quantitatively analyzed by measurement of a signal intensity obtained attributed to a specific binding reaction between the analyte and a specific binding substance. However, in a case where an excessive amount of the analyte is present with respect to the specific binding substance, that is, an analyte not specifically bound to the specific binding substance is present in the reaction system, the signal intensity obtained attributed to the specific binding reaction does not reflect the amount of the analyte in the sample.

In chromatography, for example, an analyte specifically bound to a labeled specific binding substance and an analyte not specifically bound to the labeled specific binding substance compete to cause a specific binding reaction with a specific binding substance immobilized on a carrier, which results in reduction in signal intensity attributed to the reaction contributed to by a labeling material. As thus described, there are some possibilities in the specific binding analysis method that the amount of the analyte whose concentration is high in the sample is wrongly determined to be small.

Hence a method of diluting a sample to a concentration range which is not influenced by the prozone phenomenon has been widely used, but this method has rendered a signal intensity reduced and measurement steps complicated. Also proposed has been a method of adding a specific binding substance, capable of specifically binding to an analyte, plural times to cause reactions so as to confirm whether or not the prozone phenomenon has occurred; however, this method renders measurement steps complicated.

In clinical examinations conducted employing the specific binding analysis method, examination items differ with purposes and, as the case may be, an examination by plural items using just one clinical sample (multi-term measurement) is required. While there are some cases where clinical examinations are conducted on a daily basis with the use of large-sized instruments in medical facilities and the like, the large-sized instruments generally have problems in terms of cost as well as maintenance.

Moreover, what is required in the scene of medical diagnoses including the clinical examinations and in households is a measurement device which can be operated rapidly, simply and accurately and is available at a low price. When an analyte in a sample is qualitatively or quantitatively analyzed using the specific binding analysis method, therefore, it is of necessity to read out a signal attributed to a specific binding reaction in a simple and rapid manner. Further, there has been desired a signal measurement principle comprising a small number of operation steps since low-priced measurement devices can be developed with simplification and miniaturization of measurement devices.

Accordingly, in view of the aforesaid conventional problems, an object of the present invention is to provide a specific binding analysis method which enables a qualitatively or quantitatively analysis of an analyte in a sample in a simple, rapid and accurate manner with the use of a small, simple measurement device, under little influence attributed to the concentration of the analyte in the sample.

### Disclosure of Invention

In order to solve the aforesaid problems, the present invention provides a specific binding analysis device for qualitatively or quantitatively analyzing an analyte in a sample, using a specific binding reaction between an analyte and a specific binding substance capable of specifically binding to the analyte, the device comprising: an inlet for introducing the sample; a plurality of space forming zones for temporarily retaining the sample introduced from the inlet; a plurality of reaction fields where a specific binding reaction of the sample retained in the space forming zone occurs; and an outlet for discharging the sample from the space forming zone to the reaction field, the specific binding reaction occurring under a different condition in each of the reaction fields.

It is preferable that the number of the inlets is one.

Herein, the aforesaid condition is one under which a signal intensity detected in a detection zone can be changed. This is specifically described below.

In the specific binding analysis device, it is preferable that each of the plurality of space forming zones is different in content.

It is preferable that the specific binding substance is immobilized in at least either the space forming zone or the reaction field.

It is also preferable that a characteristic of a labeling material (e.g. a particle size or a material) is different in each of the reaction fields.

Moreover, the kind of the specific binding substance is preferably different in each of the space forming zones or in each of the reaction fields. Further, an affinity of the specific binding substance is preferably different in each of the space forming zones or in each of the reaction fields. Additionally, an amount of the specific binding substance is preferably different in each of the space forming zones or in each of the reaction fields.

It is also preferable that the rate of introducing the sample from the inlet into the space forming zone is faster than the rate of discharging the sample from the outlet to the reaction field.

It is preferable that the reaction field comprises a first specific binding substance labeled with the labeling material and a second specific binding substance, and a qualitative or quantitative analysis is performed based on a signal attributed to a specific binding reaction that the first specific binding substance and the second specific binding substance are bound to one another via the analyte.

It is particularly preferable that the second specific binding substance is immobilized on the reaction field.

It is preferable that the reaction field is a developing layer capable of developing the sample due to capillarity, the detection zone is disposed on the developing layer, and the second specific binding substance is immobilized in the detection zone.

It is also preferable that the first specific binding substance is retained on the developing layer.

It is further preferable that the sample does not move between each of the plurality of developing layers. This may be realized by providing a space between each of the developing layers or forming a wall between each of the developing layers. A material with chemical resistance may constitute the wall.

It is also preferable that the signal is color, fluorescence or luminescence, and the specific binding analysis device of the present invention has a strip shape.

It is further preferable that the first specific binding substance is contained in the reaction field and the specific binding analysis device comprises a sensor for recognizing the introduction of the sample into the space forming zone.

It is preferable that at least either the first specific binding substance or the second specific binding substance is an antibody, and the labeling material is a metal sol, a dye sol, a particle containing a fluorescent substance, or a colored latex particle.

Further, the present invention relates to a specific binding analysis method for qualitatively or quantitatively analyzing an analyte in a sample, using a specific binding analysis device which comprises: an inlet for introducing the sample containing the analyte; a plurality of space forming zones for temporarily retaining the sample introduced from the inlet; a plurality of reaction fields where a specific binding reaction of the analyte in the sample retained in the space forming zone occurs; and an outlet for discharging the sample from the space forming zone to the reaction field, and is characterized in that the specific binding reaction occurs under a different condition in each of the reaction fields, and adjusting at least one selected from the group consisting of a content of the space forming zone, a dilution ratio of the sample retained in the space forming zone and the characteristic of the labeling material to analyze the analyte.

In the specific binding analysis method, it is preferable that the analyte in the sample is qualitatively or quantitatively analyzed, utilizing an amount of the sample to be introduced, which has been adjusted by adjustment of the content of the space forming zone.

It is also preferable that the introduction of the sample into the space forming zone is recognized and a standard sample is introduced into the space forming zone.

It is also preferable that the analyte in the sample is qualitatively or quantitatively analyzed by making the space forming zone carry a solution containing the first specific binding substance, and then adjusting an amount of the solution to be carried.

It is further preferable that the analyte in the sample is qualitatively or quantitatively analyzed by analyzing the product of an intensity of a signal attributed to the specific binding reaction and a magnification of the dilution.

It is preferable that the analyte in the sample is qualitatively or quantitatively analyzed by analyzing on the reaction fields a signal intensity of a reaction field where the specific binding reaction is conducted to show a signal intensity not higher than a prescribed threshold.

It is also preferable that the analyte in the sample is qualitatively or quantitatively analyzed by analyzing a signal intensity of a portion where the specific binding reaction is conducted to show the largest signal intensity among the reaction fields where the specific binding reaction is conducted to show signal intensities not higher than the threshold.

It is further preferable that the threshold is less than the minimum value of a signal intensity at an intersection which is generated when overlapping corresponding curves of an analyte concentration and a signal intensity of each of the portions where the specific binding reaction is conducted, and is also less than the minimum signal intensity among the maximum values of the signal intensities of the respective corresponding curves.

It is further preferable that when the analyte in the sample is qualitatively or quantitatively analyzed, referring to the corresponding curves of the analyte concentration and the signal intensity of the portion where the specific binding reaction is conducted to show the largest signal intensity among the reaction fields where the specific binding reaction is conducted to show signal intensities not higher than the threshold, in a case where there exist a plurality of analyte concentrations corresponding to the signal intensity of the portion where the specific binding reaction is conducted to show the largest signal intensity among the reaction fields where the specific binding reaction is conducted to show signal intensities not higher than the threshold, the smallest concentration is considered as the analyte concentration.

### Brief Description of Drawings

FIG. 1 is a schematic oblique view showing a structure of a strip comprising a single unit in the present invention.
FIG. 2 is a schematic oblique view showing a structure of a strip (a specific binding analysis device of the present invention) where a plurality of units shown in FIG. 1 are arranged.
FIG. 3 is a schematic sectional view showing the structure of the specific binding analysis device of the present invention, including analysis equipment.
FIG. 4 is a graph showing a relationship between an hCG concentration and a signal intensity in a detection zone at 520 nm, measured in Example 1.
FIG. 5 is a graph showing the relationships between the hCG concentrations and the signal intensities in a detection zones at 520 nm, measured in Example 2.
FIG. 6 is a schematic oblique view showing a structure of a strip comprising a single unit in the present invention.
FIG. 7 is a schematic oblique view showing a structure of a strip (a specific binding analysis device of the present invention) where a plurality of units are arranged, as shown in FIG. 6.
FIG. 8 is a graph showing the relationship between the hCG concentration and the signal intensity in the detection zone at 520 nm, measured in Example 3.
FIG. 9 is a graph given by overlapping graphs each showing the relationship between the hCG concentration and the signal intensity in the detection zone at 520 nm, measured in Example 4.
FIG. 10 is a graph showing the relationships between the hCG concentrations and the signal intensities in the detection zones at 520 nm, measured in Example 5.
FIG. 11 is a graph showing the relationships between the hCG concentrations and the signal intensities in the detection zones at 520 nm, measured in Example 7.
FIG. 12 is a schematic diagram of a curve drawn after analyzing a distribution status of a signal intensity in a detection zone 6 in the direction of the width of a developing layer 1.
FIG. 13 is a diagram representing the distribution statuses of the signal intensities in the detection zone 6 at 520 nm, measured five minutes after detection of the sample introduction in a strip constituted by disposing four units in parallel in Example 8.
FIG. 14 is a graph representing the relationship between the hCG concentration and the signal intensity in the detection zone 6 at 520 nm, measured five minutes after detection of the sample introduction, when the height of the curve in FIG. 13 is regarded as the signal intensity in the detection zone in each of the units.
FIG. 15 is a diagram representing the distribution statuses of the signal intensities in the detection zones 6 at 520 nm, measured five minutes after detection of introduction of samples with different concentrations in a strip constituted by disposing two units in parallel in Example 9.
FIG. 16 is a graph representing the relationships between the hCG concentrations and the signal intensities in the detection zones 6 at 520 nm, measured five minutes after detection of the sample introduction, when the height of the curve in FIG. 15 is regarded as the signal intensity in the detection zone in each of the units.
FIG. 17 is a diagram representing the distribution statuses of the signal intensities in the detection zones 6 at 520 nm, measured five minutes after detection of the sample introduction in a strip constituted by disposing two units in parallel in Example 10.
FIG. 18 is a graph representing the relationships between the hCG concentrations and the signal intensities in the detection zones 6 at 520 nm, measured five minutes after detection of the sample introduction, when the height of the curve in FIG. 17 is regarded as the signal intensity in the detection zone in each of the units.
FIG. 19 is a schematic oblique view representing a structure of a strip (a specific binding analysis device of the present invention) constituted by a plurality of arranged units and comprising a single inlet.

### Best Mode for Carrying Out the Invention

A specific binding analysis device of the present invention is a specific binding analysis device for qualitatively or quantitatively analyzing an analyte in a sample, using a specific binding reaction between an analyte and a specific binding substance capable of specifically binding to the analyte, the device comprising a plurality of units, each of which comprises: an inlet for introducing the sample; a space forming zone for temporarily retaining the sample introduced from the inlet; and an outlet for discharging the sample from the space forming zone to a reaction field where a specific binding reaction of the sample retained in the space forming zone is conducted.

Herein, the inlet may be provided separately to each of the plurality of space forming zones. In this case, the number of the space forming zones and the number of the inlets are the same. It is further preferable that just one inlet is provided to a plurality of space forming zones. In this case, the sample is supplied to all of the space forming zones from the single inlet.

It is further preferable that the specific binding analysis device in accordance with the present invention has a strip shape, and the aforesaid unit also has a strip shape. In the present description, therefore, the specific binding analysis device is sometimes referred to as a test strip. It can further be said that in the test strip of the present invention, at least two or more units are arranged, each constituted by disposing therein a space forming zone, a retention zone where a labeled first specific binding substance is retained, and a detection zone where a second specific binding substance is immobilized.

It is preferable here that the inlets, the space forming zones and the outlets, in the respective units forming the reaction fields where the specific binding reaction is conducted, are mutually separate and independent between the units. Especially when the reaction field is constituted by a developing layer, it is preferable that each of the developing layers is physically separate from one another. For example, provision of a gap or a wall between the developing layers is preferred. Thereby it becomes possible to simultaneously conduct a plurality of specific binding reactions. This also allows free adjustment of the number of the reaction measurements.

Moreover, the specific binding analysis device of the present invention is characterized in that a content of the space forming zone is different in each of the different units. It is preferable here that the content is set up in line with purposes. This enables adjustment of an introduced amount, a flow rate, and the like, of the sample in line with purposes, and even in the case of introducing the same sample, specific binding reactions under different conditions can be simultaneously observed.

For example, the adjustment of the content of the space forming zone makes adjustment of a dilution ratio possible. With this dilution ratio utilized, a prozone phenomenon which has been a problem with the conventional specific binding analysis methods can be avoided or confirmed. That is, in the present invention, previous adjustment of the content of a plurality of space forming zones enables the sample to be automatically diluted in phases so that a specific binding analysis reaction can be conducted at an analyte concentration not influenced by the prozone phenomenon. Further, it is possible to confirm whether there exists the range of a concentration at which the prozone phenomenon is occurring by observation and comparison of the results of the specific binding reactions of the sample having been diluted in phases.

Since the space forming zone temporarily retains the sample, the adjustment of the content of the space forming zone allows adjustment of the amount of the sample to be introduced. In the present invention, therefore, it is possible to accurately and automatically collect and introduce a certain amount of the sample into the specific binding analysis device without the need for using such quantitative-collecting device as a dispenser or a syringe to collect a sample and introduce it into the specific binding analysis device. It is thus possible to qualitatively or quantitatively analyze the analyte in the sample in a simple and accurate manner, without requiring an advanced device or operation.

Further, the specific binding analysis device of the present invention is characterized in that a standard sample is introduced into the space forming zone. The examples of the standard sample may include a standard substance for determination of a calibration curve to be used by the analysis device and a standard substance for quality assurance of the analysis device. The quality of the analysis device is assured by means of response of the analysis device itself, a reaction characteristic of a regent to be used in the analysis device, or the like.

As for a specific binding analysis method aiming at a quantitative analysis widely used has been a method of conducting a specific binding reaction which uses an analyte and a specific binding substance capable of specifically binding to the analyte, and also using a calibration curve for concentration conversion, which is created by previously conducting the specific binding reaction using a standard substance during preparation of the analysis device, to determine the concentration of the analyte.

In effect, however, a plurality of calibration curves are prepared during preparation of the analysis device for the reasons of differences between each of the lots of the analysis devices, changes in performance thereof during storage, and the like. This necessitates preparation of an analysis device for determination of a calibration curve, as well as a standard substance for determination of a calibration curve, to be used in each of the lots so that a calibration curve for concentration conversion to be applied to each of the analysis devices can be determined.

In the present invention, it is possible to confirm as to whether the analysis device is in a state capable of being provided the quality assurance, by automatically creating a dilution system by the use of a standard substance with a known concentration conducting a specific binding reaction, and then comparing prepared reaction curves, for example. In the present invention, determination of the calibration curve and the quality assurance become possible by conducting the specific binding reaction using the standard substance as the standard sample, and then measuring the response of the analysis device, the reaction characteristic of the reagent or the like.

The analyte in the present invention may be any one so long as a specific binding substance capable of specifically binding thereto exists, and the examples may include various proteins, which can function as an antibody or antigen, polypeptides, glycoproteins, polysaccharides, complex glycolipids, nucleic acids, effector molecules, receptor molecules, enzymes and inhibitors. More specific examples may include: tumor markers such as α-fetoprotein, carcinoembryonic antigen (CEA), CA 125 and CA 19-9; various proteins, glycoproteins or complex glycolipids such as β2-microglobulin (β2m) and ferritin; various hormones such as estradiol (E2), estriol (E3), human chorionic gonadotropin (hCG), luteinizing hormone (LH) and human placental lactogen (hPL); various virus-associated antigens or virus-associated antibodies such as HBs antigen, HBs antibody, HBc antigen, HBc antibody, HCV antibody and HIV antibody; various allergens and IgE antibodies thereto; narcotic drugs, medical drugs and metabolites thereof; and virus-associated and tumor-associated nucleic acids having a polynucleotide sequence.

The sample in the present invention may be a liquid suspected of containing an analyte, and the examples may include urine, blood serum, blood plasma, whole blood, saliva, lacrimal fluid, spinal fluid and secretion from papillae; however, the sample may also be one prepared by suspending or dissolving a solid substance, a gel substance or sol substance of mucus, human body tissue, cell or the like, in a liquid such as a buffer solution, an excessct solution or a dissolved solution.

The specific binding substance in the present invention may be any substance capable of specifically binding to the analytes, and the examples may include antibodies, antigens, glycoproteins, polysaccharides, complex glycolipids, nucleic acids, effector molecules, receptor molecules, enzymes and inhibitors.

Further, in the specific binding analysis method using the specific binding analysis device in accordance with the present invention, in a step of causing a reaction between an analyte and a specific binding substance capable of specifically binding to the analyte, it is preferable that a first specific binding substance labeled with a labeling material and a second specific binding substance are employed and the labeled first specific binding substance and the second specific binding substance are bound to one another, via the analyte.

The specific binding analysis method using the specific binding analysis device in the present invention is characterized in that the analyte in the sample is qualitatively or quantitatively analyzed by adjusting an amount of a solution containing the first specific binding substance labeled with the labeling material. The adjustment of the amount of the solution containing the specific binding substance labeled with the labeling material enables dilution of the sample in the step of causing the reaction between the analyte and the specific binding substance capable of specifically binding to the analyte, and utilization of a dilution ratio thus obtained enables avoidance and confirmation of the prozone phenomenon which has been a problem with the conventional specific binding analysis methods.

In the present invention, it is also possible to qualitatively or quantitatively analyze the analyte in the sample by adjusting the characteristic of the labeling material in each of the reaction fields where the specific binding reaction is conducted. The characteristic of the labeling material for example is a particle size or a material of the labeling material. With the characteristic of the labeling material changed, the kind as well as an intensity of a signal that can be read out from the labeling material also change. It is thus possible to chose a reaction field capable of sending an appropriate signal for analysis of the analyte concentration in the sample, whereby the prozone phenomenon which has been a problem with the conventional specific binding analysis methods can be avoided or confirmed, without diluting the sample. Further, adjustment of an amount of the first specific binding substance labeled with the labeling material in each of the reaction fields where the specific binding reaction is conducted, by utilizing the characteristic of the labeling material, makes it possible to adjust a component ratio between the analyte and the specific binding substance which are capable of participating in the specific binding reaction, whereby the specific binding reaction can be observed under conditions where no prozone phenomenon occurs, without diluting the sample. In this manner, the prozone phenomenon which has been a problem with the conventional specific binding analysis methods can be avoided or confirmed.

In the present invention, the analyte in the sample may be qualitatively or quantitatively analyzed by adjusting the first specific binding substance labeled with the labeling material and/or the second specific binding substance in each of the reaction fields where the specific binding reaction is conducted. Disposition of specific binding substances capable of specifically binding to different analytes in the respective reaction fields where the specific binding reaction is conducted makes it possible to adjust an analyte to be detected in each of the reaction fields where the specific binding reaction is conducted. Even in a case where a plurality of analytes are present in the sample, therefore, disposition of a specific binding substance capable of specifically binding to a certain analyte in line with a purpose in each of the reaction fields where the specific binding reaction is conducted makes it possible to simultaneously detect a plurality of analytes and also to correspond to the multi-item measurement which has been required in the clinical field.

In the specific binding analysis device in the present invention, the solution containing the first specific binding substance labeled with the labeling material may be sealed into the space forming zone. Thereby, if the sample is introduced from the inlet, the specific binding reaction between the analyte in the sample and the first specific binding substance labeled with the labeling material can be conducted while the sample is retained in the space forming zone. Further, adjustment of an amount of the solution containing the first specific binding substance labeled with the labeling material to be sealed into the space forming zone enables automatic dilution of the sample in phases so that the prozone phenomenon which has been a problem with the conventional specific binding analysis method can be avoided or confirmed.

It is to be noted that in the specific binding analysis device in the present invention, when the contents of the respective space forming zones are equal, the first specific binding substance labeled with the labeling material may not be sealed in the space forming zone. For example, when the amount of the first specific binding substance labeled with the labeling material is adjusted in each of the reaction fields where the specific binding reaction is conducted, it becomes possible to adjust the component ratio between the analyte and the specific binding substance which are capable of participating in the specific binding reaction in each of the reaction fields where the specific binding reaction is conducted so that the specific binding reaction can be observed under conditions where no prozone phenomenon occurs, without diluting the sample, and the prozone phenomenon which has been a problem with the conventional specific binding analysis methods can be avoided or confirmed.

In addition, disposition of first specific binding substances labeled with the labeling material, having different affinities in the respective reaction fields where the specific binding reaction is conducted, makes the specific binding reaction between the analyte and the specific binding substance controllable, by utilizing the affinity difference, in each of the reaction fields where the specific binding reaction is conducted so that the specific binding reaction can be observed under conditions where no prozone phenomenon occurs, without diluting the sample, and the prozone phenomenon which has been a problem with the conventional specific binding analysis methods can be avoided or confirmed.

Further, disposition of specific binding substances, which are capable of specifically binding to the same analyte but have different characteristics, in the respective reaction fields where the specific binding reaction is conducted, renders the specific binding reaction between the analyte and the specific binding substance controllable, by utilizing the characteristic difference, in each of the reaction fields where the specific binding reaction is conducted. For example, when specific binding substances with different affinities are disposed, the specific binding reaction between the analyte and the specific binding substance can be controlled by utilizing the affinity difference in each of the reaction fields where the specific binding reaction is conducted, and thus the specific binding reaction can be observed under conditions where no prozone phenomenon occurs, without diluting the sample. That is, the prozone phenomenon which has been a problem with the conventional specific binding analysis methods can be avoided or confirmed. It should be noted that the specific binding reaction can be controlled simply by replacement of the first specific binding substance with the second specific binding substance in the combination thereof.

Moreover, in the specific binding analysis device of the present invention, it is preferable that the unit comprises a detection zone for detecting a signal obtained attributed to the specific binding reaction and the second specific binding substance is substantially immobilized in the detection zone. Herein, the second specific binding substance is immobilized in the detection zone so as not to move freely even when the developing layer constituting the reaction field is in a wet state. In such a manner, an unreacted substance is washed off along with the sample flow, thereby enabling detection of the signal attributed to the specific binding reaction in the detection zone without conducting an operation of separating the unreacted substance. It is to be noted that the device of the present invention may comprise a plurality of detection zones.

The developing layer in the present invention is the reaction field where the analyte in the sample and the first specific binding substance labeled with the labeling material develop. The examples thereof may include a porous carrier and a gel carrier. Among them, nitrocellulose is most preferable. This material is superior to other developing layer materials such as paper because it is inherently capable of binding to protein without previous sensitization. When directly applied to nitrocellulose, a specific binding substance such as an antibody can be reliably immobilized, without requiring any chemical treatment that may suppress the specific binding capability of the specific binding substance. When paper is used as the developing layer material, for example, the immobilization of the antibody necessitates a chemical binding using CNBr, carbonyldiimidazole, tressil chloride or the like.

Furthermore, nitrocellulose is available in various pore sizes so that the developing layer material can be readily selected according to conditions such as a flow rate of the sample. It is desirable that strength of a nitrocellulose sheet, when handled, is improved by attaching a plastic sheet or the like onto the back of the sheet. This can be readily made by forming a thin film of nitrocellulose on a reinforcing material sheet.

After the antibody is immobilized in the detection zone, a portion, other than the portion in which the antibody is immobilized, is preferably blocked to reduce non-specific absorption to the developing layer. The blocking may be achieved by application of protein (e.g., bovine serum albumin and milk protein), polyvinylalcohol or ethanolamine, or a combination thereof.

The space forming zone in the present invention may be one capable of forming a space for temporality retaining the sample, and further, it serves to accurately and automatically collect and introduce a certain amount of the sample, depending on the volume thereof, into the specific binding analysis device and also serves as an anti-pollution protective material when the device is handled after the introduction of the sample. For example, it can be constituted using a synthetic resin material such as ABS, polystyrene or poly vinyl chloride, or a solution-impermeable material such as metal or glass.

In the present invention, the inlet is one for introducing the sample and the outlet is one for discharging the sample retained in the space forming zone to the reaction field where the specific binding reaction is conducted. It is preferable here that the rate of introducing the sample from the inlet into the space forming zone is sufficiently faster than the rate of discharging the sample from the outlet to the reaction field where the specific binding reaction is conducted. Thereby, the sample brought into contact with the inlet flows into the space forming zone due to capillarity phenomenon, and the amount thereof to be introduced becomes substantially equivalent to the content of the space forming zone. It is therefore possible to accurately and automatically collect and introduce a certain amount of the sample into the specific binding analysis device. Further, temporary retention of the sample in the space forming zone after the sample flows in becomes possible so that, even in the case of sealing a reagent into the space forming zone, the time taken for the reaction between the sample and the reagent can be secured.

The signal in the present invention may be one which is generated by a reaction contributed to by the labeling material and can be detected, and the examples may include fluorescence measurable with a fluorescent photometer, luminescence measurable with a luminescent photometer and exhibited color measurable by visual determination and a color-difference meter. In this case, an intensity of reflected light, an intensity of fluorescence or luminescence generated in the detection zone, or the like, is detected in the detection zone.

In one of the embodiments of the present invention, a sample is introduced into a test strip connected to a signal intensity measurement device, or a test strip with a sample introduced therein is connected to the signal intensity measurement device, and then the degree of modulation of a signal in a detection zone is measured by naked-eye visual observation or with a suitable external measurement device according to the property of the signal. Between the aforesaid measurements more preferred is one where the test strip is connected to the signal intensity measurement device and the sample is then introduced into the test strip to detect the signal.

In one of the embodiments of the present invention, it is preferable that signals attributed to a plurality of specific binding reactions, sent from the respective reaction fields where the specific binding reaction is conducted, are distinguished due to the respective reaction fields where the specific binding reaction is conducted and successively read out so that the analyte in the sample is qualitatively or quantitatively analyzed. This operation makes it possible to reduce the time required for the step of reading out the signal even when a plurality of specific binding reactions are conducted, and to correspond to clinical examination devices as well as sensors for the domestic use which require rapid determination of results.

It is also preferable that the reaction field where the specific binding reaction is conducted and/or a device for reading out a signal move in a certain direction so that signals sent from the respective reaction fields where the specific binding reaction is conducted are successively read out. For example, in the case of using a strip where a plurality of detection zones, capable of reading out a signal, are arranged in parallel, scanning of the signal intensity measurement device in the direction along the detection zones allows successive measurement of signals obtained from a plurality of specific binding reactions. Even in a case where, reversely, the signal intensity measurement device is immobilized while the strip is scanned, a similar effect can be expected to be obtained. This operation can simplify the device required for the signal intensity measurement and can also make the device smaller and less expensive.

Moreover, when the specific binding analysis device of the present invention is used, a step of recognizing the introduction of the sample from the inlet is preferably performed. For example employed is a start-of-measurement detector, or the like, for detecting the introduction of the sample and the start of the measurement by using a difference between a conductivity of the developing layer in a dry state before the sample introduction and a conductivity of the developing layer in a wet state changed from the dry state due to the sample introduction. It is preferable to start measuring the measurement time on the basis of the time at which the introduction of the sample from the inlet is recognized. This operation enables accurate control of the detection time of signal, i.e., the time elapsed between the sample introduction and the signal intensity measurement, and also enables reduction in error caused by measurement manipulation.

It is further preferable that the labeled first specific binding substance is contained in the developing layer. In such a manner, the sample introduced into the test strip reacts with the labeled first specific binding substance when developing along the developing layer so that a step of causing a reaction between the sample and the labeled first specific binding substance can be simplified.

It is further preferable that the labeled first specific binding substance is in a dry state. Herewith, when the developing layer is in the dry state, the labeled first specific binding substance is retained in the retention zone on the developing layer; when the developing layer gets wet caused by the sample introduction or the like, the labeled first specific binding substance can freely move within the developing layer. The developing layer material is made band-shaped or sheet-shaped, and the reagent is applied onto spatially separate regions on the developing layer and is made to infiltrate from the side or the end of the sheet or the band to the other side at the time of the sample introduction.

It should be noted that the sample may be introduced into the strip after being previously reacted with the labeled first specific binding substance in the outside of the strip. In such a case, the labeled first specific binding substance may not be contained in the developing layer or the space forming zone.

The first specific binding substance and the second specific binding substance in the present invention may be substances which are capable of specifically binding to analytes, and the examples thereof may include antibodies, antigens, glycoproteins, polysaccharides, complex glycolipids, nucleic acids, effector molecules, receptor molecules, enzymes and inhibitors.

Among them, at least either the first specific binding substance or the second specific binding substance is preferably an antibody, and more preferably a monoclonal antibody, from the viewpoint of high specificity. It is further preferable that the first specific binding substance and the second specific binding substance have specificities against different epitopes of the analyte. When the analyte has a plurality of same epitopes, the same specific binding substances may be used.

The labeling material in the present invention may be an arbitrary substance whose presence can be readily detected. The preferable one is a direct label, namely a material visible with the naked eye in a natural state, or readily visible with the use of an optical filter and/or by the use of a method of promoting fluorescence by adding a stimulation such as an ultraviolet ray or the like. Since the direct label is capable of generating a signal which is detectable, without addition of another reagent, analysis results can be obtained in a moment of time, and further, the direct label can be readily used in an analysis device stored in a dry state because of the strong and stable properties thereof.

As the direct label particularly preferred are fine colored particles such as dye sols, metal sols, particles containing a fluorescent substance and colored latex particles. Employment of such particles enables concentration of the label in a small area or volume, which forms a region thickly colored to generate a signal which is readily detectable. Among them most preferred are colloidal gold particles. This evaluation of a signal can be conducted by visual estimation or, if required, by using apparatus.

In the meantime, indirect labels such as enzymes like alkaline phsphotase, horseradish peroxidase, etc. can be employed. Reasonably, however, the indirect label is inferior to the direct label in terms of simplicity since detection of a visible signal usually requires addition of one or more development reagents such as a substrate. In such a case of adding the development reagent, the reagent may be contained in a porous development layer material so as to dissolve or disperse in the sample. Another method available may be to mix the sample with the development reagent beforehand, and then introduce the resulting mixture into the test strip after occurrence of the specific binding reaction.

It is to be noted that the labeling material and the specific binding substance can be bound to one another by covalent bonding or hydrophobic bonding, as circumstances demand.

In the test strip used in a preferable embodiment of the specific binding analysis device of the present invention, when the analyte is present in the sample, a sandwich reaction occurs that the labeled first specific binding substance and the second specific binding substance immobilized in the detection zone are bound to one another via the analyte, and in the detection zone, the labeled first specific binding substance is bound. An intensity of a signal from the labeled first specific binding substance as thus bound in the detection zone is measured, and then the analyte in the sample is qualitatively or quantitatively analyzed based on the measured signal intensity.

Herein, the labeled first specific binding substance may move as the sample flows toward the detection zone, and it is preferable that the sample keeps flowing even after passing through the detection zone. For this purpose, a sufficient amount of the sample is introduced into the test strip so that an excess of the labeled first specific binding substance, which is not capable of participating in the specific binding reaction in the detection zone, is washed off from the detection zone by the sample flow through the detection zone. An absorption zone may be provided at the end of the developing layer material. The material for the absorption zone may be any material having enough an absorptive property to wash off an unbound composition out of the detection zone, which may be exemplified by the glass fiber filter paper GA-200 (manufactured by TOYO KABUSHIKI KAISHA).

It is further preferable that the product of a signal intensity and a dilution magnification is used to qualitatively or quantitatively analyze the analyte in the sample. In the present invention, the corresponding relationship between the analyte concentration and the signal intensity with regard to a standard substance with a known concentration is previously assayed to create a calibration curve and, for example, from a signal intensity, measured in terms of a sample to be detected which has been automatically diluted to have an analyte concentration not influenced by the prozone phenomenon, the analyte concentration contained in the sample to be detected can be determined by the use of the calibration curve. In such a case, the product of the dilution ratio and the analyte concentration obtained using the calibration curve is the analyte concentration in the sample.

Further, the specific binding analysis method using the specific binding analysis device of the present invention is characterized in that the analyte in the sample is qualitatively or quantitatively analyzed by analyzing a signal intensity of a reaction field where the specific binding reaction is conducted to show a signal intensity not higher than a certain threshold. It is preferable here that the threshold is less than the signal intensity shown by the analyte with such a concentration as causing no prozone phenomenon. Herewith, it is possible to qualitatively or quantitatively analyze the analyte while avoiding the prozone phenomenon.

It is further preferable that the analyte in the sample is qualitatively or quantitatively analyzed by analyzing a signal intensity of a reaction field where the specific binding reaction is conducted to show the largest signal intensity among the reaction fields where the specific binding reaction is conducted to show signal intensities not higher than a certain threshold. In the present invention, with regard to a standard substance with a known concentration, the corresponding relationship between the analyte concentration and the signal intensity in the detection zone is previously assayed to create a calibration curve. When the analyte is quantitatively analyzed using the calibration curve, a quantitative property is improved by using a calibration curve having a large inclination, namely a large value given by dividing the signal intensity by the concentration, in the straight line region where the prozone phenomenon is not occurring.

Further, in the specific binding analysis method using the specific binding analysis device of the present invention, the analyte is qualitatively or quantitatively analyzed by comparing signal intensities of a plurality of reaction fields where the specific binding reaction is conducted; when comparing the straight line regions of the calibration curves among the reaction fields where the specific binding reaction is conducted to show signal intensities not higher than a certain threshold, a larger signal intensity is observed on a more-steeply-inclined calibration curve. Hence it becomes possible to qualitatively or quantitatively analyze the analyte in the sample in a more accurate manner by analyzing the signal intensity of the reaction field where the specific binding reaction is conducted to show the largest signal intensity among the reaction fields where the specific binding reaction is conducted to show signal intensities not higher than a predetermined threshold.

Moreover, the present invention is characterized in that, when the analyte in the sample is qualitatively or quantitatively analyzed, referring to the corresponding curves of the analyte concentration and the signal intensity of the reaction field where the specific binding reaction is conducted to show the largest signal intensity among the reaction fields where the specific binding reaction is conducted to show signal intensities not higher than the threshold, in a case where there exist a plurality of analyte concentrations corresponding to the signal intensity of the reaction field where the specific binding reaction is conducted to show the largest signal intensity among the reaction fields where the specific binding reaction is conducted to show signal intensities not higher than the threshold, the smallest concentration is considered as the analyte concentration. Further, the threshold is less than the minimum value of a signal intensity at an intersection which is generated when overlapping the corresponding curves of the analyte concentration and the signal intensity of the respective reaction fields where the specific binding reaction is conducted, and is also less than the minimum signal intensity among the maximum values of the signal intensities of the respective corresponding curves.

In the present invention, with regard to a standard substance with a known concentration, the corresponding relationship between the analyte concentration and the signal intensity in the detection zone is previously assayed to create a calibration curve.

In the specific binding analysis method using the specific binding analysis device of the present invention, the analyte is qualitatively or quantitatively analyzed by comparing signal intensities of a plurality of reaction fields where the specific binding reaction is conducted; when overlapping calibration curves of the respective reaction fields where the specific binding reaction is conducted, the possibility may arise where an intersection is generated between the calibration curves, if creation of a calibration curve accompanied by the assay where prozone phenomenon is present. A value less than the minimum values of signal intensities of these intersections and also less than the minimum signal intensity among the maximum values of the signal intensities of the respective calibration curves is set up as a threshold, reaction fields where the specific binding reaction is conducted to show signal intensities not higher than the threshold are selected, a reaction field to show the maximum signal intensity is selected by comparing the signal intensities among those reaction fields, and then the signal intensity of the selected reaction field is analyzed, to qualitatively or quantitatively analyze the analyte in the sample.

In the present invention, since the analyte in the sample is qualitatively or quantitatively analyzed by the use of corresponding curves of a plurality of reaction fields where the specific binding reaction is conducted, the range of the qualitative or quantitative analysis is extended as compared to that in the conventional specific binding analysis method using a single corresponding curve. When the analyte concentration in the sample is less than the minimum value of an analyte concentration at an intersection generated when overlapping the calibration curves of the respective reaction fields where the specific binding reaction is conducted, as comparing signal intensities of reaction fields, where the sample is introduced and the specific binding reaction is conducted, the analyte in the sample shows a larger signal intensity in a reaction field whose calibration curve is more steeply inclined in the straight line region, where the prozone phenomenon is not occurring. Therefore, when the magnitude relationship among the signal intensities and the inclinations of the calibration curves in the straight line region where the prozone phenomenon is not occurring, in each of the reaction fields where the specific binding reaction is conducted, is in the aforesaid state, in a case where there exist a plurality of analyte concentrations corresponding to a signal intensity of the reaction field selected as the reaction field for qualitatively or quantitatively analyzing the analyte in the sample, the smallest concentration among the corresponding concentrations is the analyte concentration in the sample.

On the other hand, when the analyte concentration in the sample is not less than the minimum value of the analyte concentration at the intersection generated when overlapping the calibration curves of the respective reaction fields where the specific binding reaction is conducted, the magnitude relationship among the signal intensities shown by the analyte in the sample in the reaction fields, where the sample is introduced and the specific binding reaction is conducted, do not correspond to the magnitude relationship among the inclinations of the calibration curves in the straight line regions where the prozone phenomenon is not occurring. The signal intensity and the inclination of the calibration curve in the straight line region where the prozone phenomenon is not occurring is in such a state, the specific binding analysis device in the present invention judges the analyte concentration in the sample as being out of the scope of the qualitatively or quantitatively analysis. In such a case, a sample further diluted is employed or a specific binding analysis device capable of corresponding to an analyte with a high concentration is used, to qualitatively or quantitatively analyze the analyte in the sample.

In the present invention, since it is possible to qualitatively or quantitatively analyze the analyte in the sample in a case where the analyte concentration is less than the minimum value of the analyte concentration at the intersection generated when overlapping the calibration curves of the respective reaction fields where the specific binding reaction is conducted, the range of qualitatively or quantitatively analyzing the analyte is extended by preparing a device with a large minimum value of the analyte concentration at this intersection. Further, in the case of using an analyte where it matters whether or not the analyte is not less than a normal value in a certain range, such as some kind of clinical marker, it is clinically useful to set up a value adjacent to the normal value as a threshold since it can be a criterion of decision as to whether or not an abnormal value is shown.

When an intersection is not generated between calibration curves, the analyte is qualitatively or quantitatively analyzed by analyzing a signal intensity of a reaction field whose calibration curve in the straight line region is most steeply inclined among reaction fields where the specific binding reaction is conducted to show signal intensities not higher than a certain threshold. The threshold in this case is less than the maximum value of the signal intensity whose calibration curve in the straight line region, where the prozone phenomenon is not occurring, is most steeply inclined among calibration curves of reaction fields where the specific binding reaction is conducted.

As thus described, it is possible to qualitatively or quantitatively analyze the analyte in the sample, using the present invention, without being influenced by the prozone phenomenon which has been a problem with the conventional specific binding reaction analysis, by comparing the magnitude relationship among the signal intensities of the respective reaction fields where the specific binding reaction is conducted and the magnitude relationship among the inclinations of the calibration curves in the straight line regions where the prozone phenomenon is not occurring, and comparing the signal intensities of the respective reaction fields where the specific binding reaction is conducted to show signal intensities not higher than a threshold, set up by utilizing the present invention, and to select the reaction field to show the maximum signal intensity, which is used for analysis.

### Embodiment 1

Embodiments of the present invention are specifically described here by reference to the drawings. However, the present invention is not limited thereto.

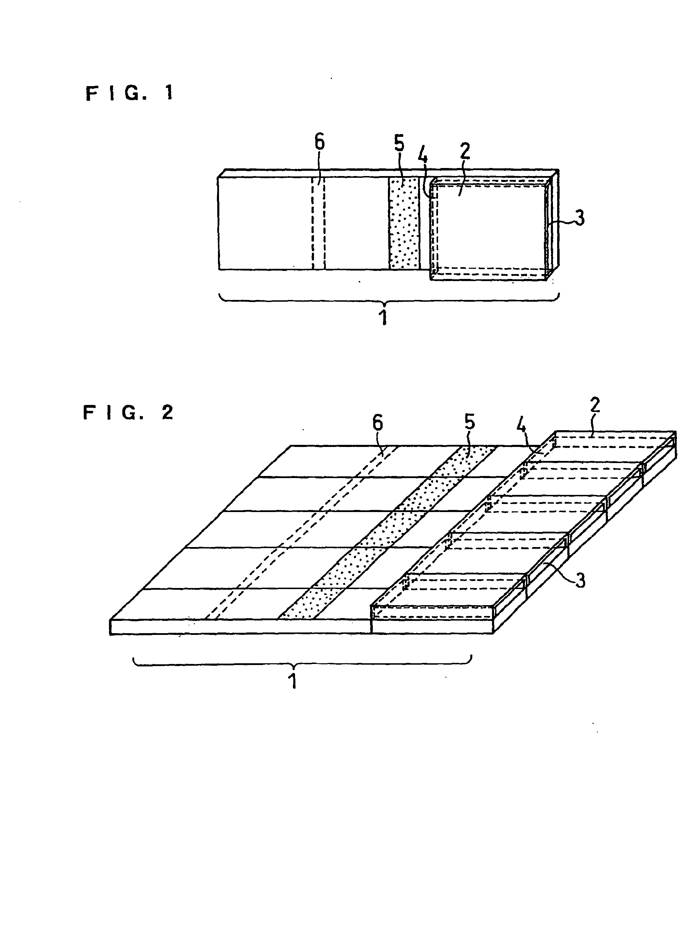

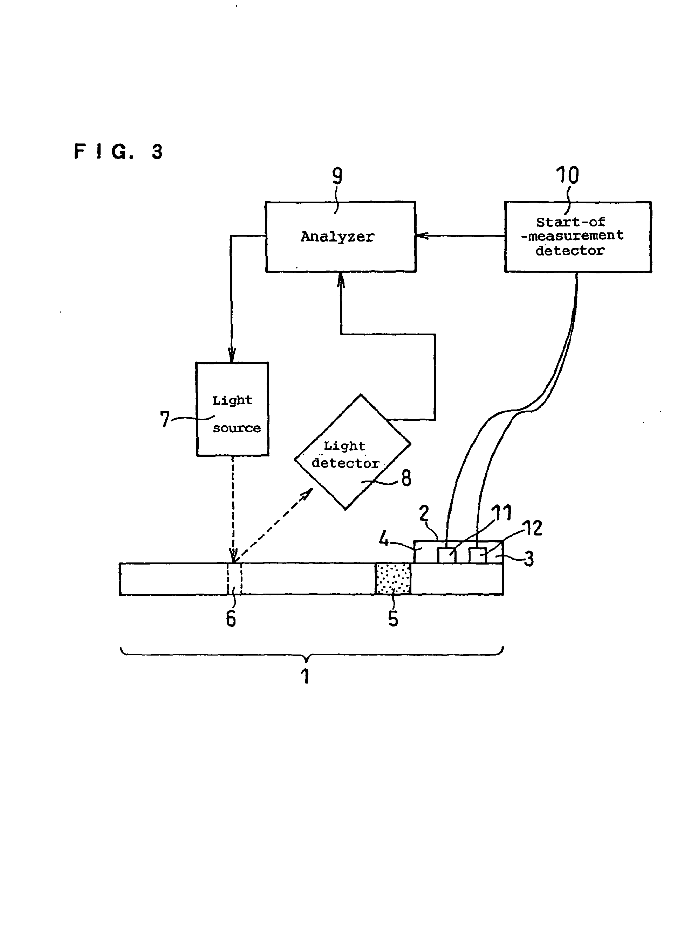

FIG. 1 is a schematic oblique view of a single unit constituting a test strip as a specific binding analysis device in accordance with the present embodiment, and FIG. 2 is a schematic oblique view of a test strip in accordance with the present invention in which a plurality of units, shown in FIG. 1, are linked to one another. Further, FIG. 3 is a schematic sectional view showing a structure of an analysis device and a test strip of the present invention.

As a representative example described is a case where hCG is used as an analyte. A monoclonal antibody to hCG, which is capable of participating in a sandwich reaction with hCG, is used as a first specific binding substance and a second specific binding substance. And colloidal gold is used as a labeling material (direct label).

As shown in FIG. 3, the strip is constituted by a developing layer 1 on which a space forming zone 2 with an equivalent content is disposed. Further, on the developing layer 1, the anti-hCG monoclonal antibody as the first specific binding substance is retained in a retention zone 5 in the state of being labeled with colloidal gold, and the anti-hCG monoclonal antibody as the second specific binding substance is immobilized in a detection zone 6; on the upper face of the developing layer 1, a first electrode 11 and a second electrode 12 are disposed. Further, an analysis device is constituted by a light source 7, a light detector 8, an analyzer 9 and a start-of-measurement detector 10 as a means to recognize introduction of a sample into the strip.

The detection zone 6 is irradiated with light having a prescribed wavelength (e.g. 520 nm) from the light source 7, and the reflected light is detected with the light detector 8 and then analyzed with the analyzer 9, to measure an absorption in the detection zone 6. As the measurement wavelength selected may be one suitable for the sample or exhibition of color of the labeling material in the detection zone 6.

A sample containing hCG is introduced into the test strip. When the sample is brought into contact with an inlet 3, a certain amount of the sample flows into the space forming zone 2 due to capillarity phenomenon. With the introduction of the sample, the developing layer 1 in a dry state changes to be in a wet state, and a conductivity of the developing layer 1 thus changes. Using the first electrode 11 and the second electrode 12, the conductivity of the developing layer 1 in a dry state is previously measured prior to the sample introduction and then the conductivity is monitored during the sample introduction so that the start-of-measurement detector 10 can detect the sample introduction from the change in conductivity of the developing layer 1. Once the sample introduction is detected, the information on this detection is sent from the start-of-measurement detector 10 to the analyzer 9, which starts calculation of the measurement time on the assumption that the time at which the sample introduction is detected is zero.

The analyzer 9 has the function of controlling the light source 7 and the light detector 8 so as to automatically measure the absorption in the detection zone 6 at a previously predetermined time. The sample temporarily retained in the space forming zone 2 is discharged out of an outlet 4, develops along the developing layer 1 and arrives at the retention zone 5, where hCG in the sample and the anti-hCG monoclonal antibody bound to colloidal gold form a labeled antigen-antibody complexes. Subsequently, upon arrival of the sample at the detection zone 6, the labeled antigen-antibody complexes are bound to the immobilized anti-hCG monoclonal antibody so that the labeled anti-hCG monoclonal antibody is trapped in the detection zone 6 to exhibit color.

The absorption of the labeling material in the detection zone 6 reflects an amount of hCG in the sample. In the analysis example of the present invention, a sampling inspection is previously conducted at the time of preparation of the analysis devices where the absorptions in the detection zones 6 are measured using a standard substance, and further, with the use of the analysis device from the same lot, the absorptions in the detection zone 6 measured with respect to the respective hCG concentrations are plotted to give a calibration curve. Herewith, a plurality of calibration curves for concentration conversion are prepared during preparation of the analysis devices, and then, when using the analysis devices, measurements are conducted using the standard sample, and from the absorption in the detection zone 6 shown by the standard sample, a calibration curve for concentration conversion to be applied to each of the analysis devices is determined. With this calibration curve utilized, the hCG concentration in the sample introduced to the developing layer 1 can be calculated from the absorption in the detection zone 6.

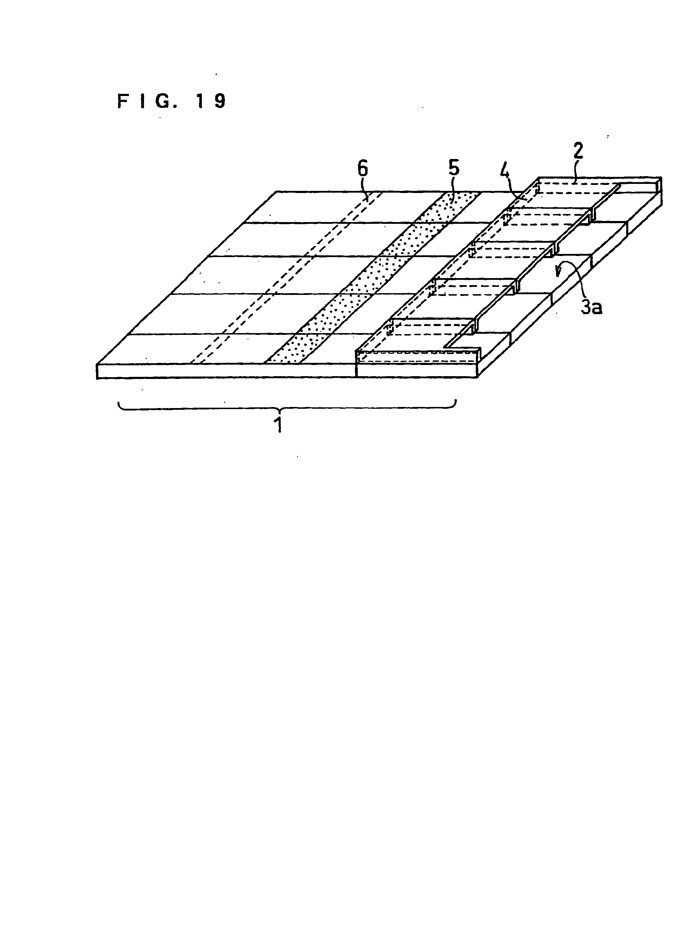

Herein, FIG. 19 shows a schematic oblique view of a strip (a specific binding analysis device) in accordance with the present invention, comprising a single inlet. In the present invention, as shown in FIG. 19, the strip may be constituted by a plurality of units and just a single inlet. In the strip shown in FIG. 2, the sample needs to be introduced separately into each inlet of a plurality of units; however, in the strip shown in FIG. 19, the sample can be introduced into each of the units only by one-time introduction of the sample into one inlet(3a) due to capillary phenomenon. It should be noted that the constituents shown by the reference numbers in FIG. 19 are the same as those shown by the reference numbers in FIG. 1.

### Embodiment 2

Embodiment 2 of the present invention also has the structures shown in FIGS. 1 to 3.

As the present embodiment described is a case where the analyte is hCG, each of the first specific binding substance and the second specific binding substance is the monoclonal antibody to hCG, which is capable of participating in a sandwich reaction with hCG, and colloidal gold is used as the labeling material for direct labeling.

On the developing layer 1, the anti-hCG monoclonal antibody as the first specific binding substance is retained in a retention zone 5 in the state of being labeled with colloidal gold, and the anti-hCG monoclonal antibody as the second specific binding substance is immobilized in a detection zone 6; on the upper face of the developing layer 1, a first electrode 11 and a second electrode 12 are disposed.

The strip is constituted by the developing layers 1, on which the space forming zones 2 are equal in volume while the retention zones 5 are different in retained amount of the anti-hCG monoclonal antibody, as the first specific binding substance, labeled with colloidal gold. The analysis device is constituted by a light source 7, a light detector 8, an analyzer 9 and a start-of-measurement detector 10 for recognizing introduction of a sample into the test strip.

The detection zone 6 is irradiated with light having a prescribed wavelength (e.g. 520 nm) from the light source 7, and the reflected light is detected with the light detector 8 and then analyzed with the analyzer 9, to measure an absorption in the detection zone 6. As the measurement wavelength selected may be one suitable for the sample or exhibition of color of the labeling material in the detection zone 6.

A sample containing hCG is introduced into the test strip. When the sample is brought into contact with an inlet 3, a certain amount of the sample flows into the space forming zone 2 due to capillarity phenomenon. With the introduction of the sample, the developing layer 1 in a dry state changes to be in a wet state, and a conductivity of the developing layer 1 thus changes. Using the first electrode 11 and the second electrode 12, the conductivity of the developing layer 1 in a dry state is previously measured prior to the sample introduction and then the conductivity is monitored during the sample introduction so that the start-of-measurement detector 10 can detect the sample introduction from the change in conductivity of the developing layer 1. Once the sample introduction is detected, the information on this detection is sent from the start-of-measurement detector 10 to the analyzer 9, which starts calculation of the measurement time on the assumption that the time at which the sample introduction is detected is zero.

The analyzer 9 has the function of controlling the light source 7 and the light detector 8 so as to automatically measure the absorption in the detection zone 6 at a previously predetermined time. The sample temporarily retained in the space forming zone 2 is discharged out of the outlet 4, develops along the developing layer 1 and arrives at the retention zone 5, where hCG in the sample and the anti-hCG monoclonal antibody bound to colloidal gold form a labeled antigen-antibody complexes. Subsequently, upon arrival of the sample at the detection zone 6, the labeled antigen-antibody complexes are bound to the immobilized anti-hCG monoclonal antibody so that the labeled anti-hCG monoclonal antibody is trapped in the detection zone 6 to exhibit color.

The absorption of the labeling material in the detection zone 6 reflects an amount of hCG in the sample. In the analysis example of the present invention, an amount of the sample introduced to each of the developing layers 1 is equal since the space forming zone 2 on each of the developing layers 1 is equal in volume; however, the absorption in each of the detection zones 6 is different since an amount of the anti-hCG monoclonal antibody, as the first specific binding substance, labeled with colloidal gold, retained in each of the retention zones 5 is different. Accordingly, the introduction of the sample into the test strip allows automatic, simultaneous observation of reactions between the antigen and the antibody in different composition ratios.

In the present invention, it is possible to automatically adjust the composition ratio of the analyte and the specific binding substance which are capable of participating in the specific binding reaction on each of the developing layers 1, by previously adjusting the amount of the anti-hCG monoclonal antibody, as the first specific binding substance, labeled with colloidal gold, retained in the detection zone 5 on each of the developing layers 1, and it is also possible to calculate the hCG concentration in the sample introduced into the test strip by observing and comparing the results of the specific binding reactions between the analyte and the specific binding substance in different composition ratios, and by utilizing the calibration curve, from the absorption of the labeling material in the detection zone 6 on the developing layer 1 with the composition ratio not influenced by the prozone phenomenon.

### Embodiment 3

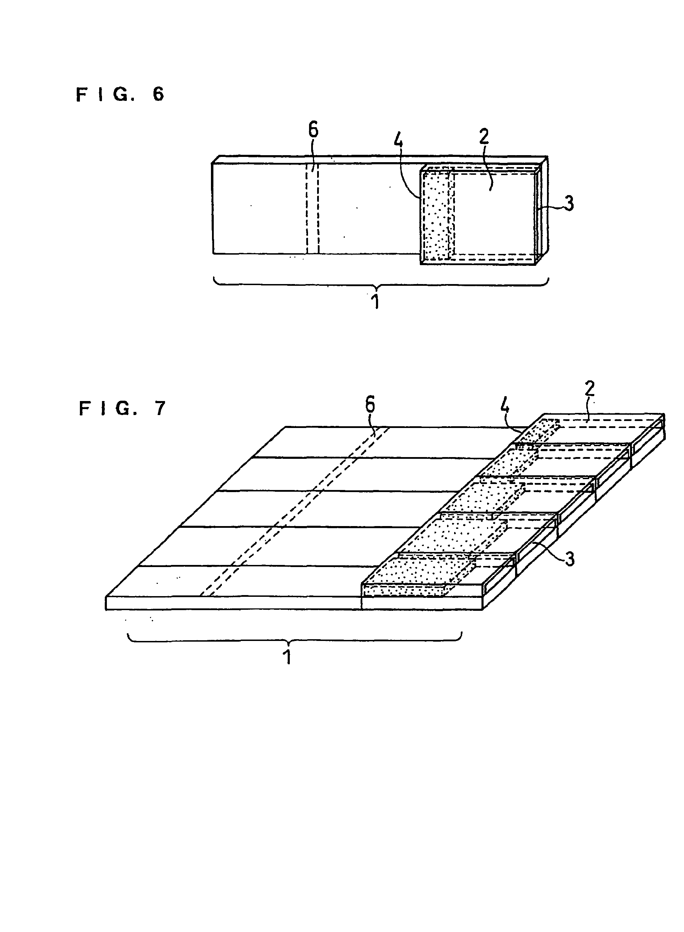

The present embodiment is described in detail by reference to FIGS. 6, 7 and 3.

FIG. 6 is a schematic oblique view of a single unit constituting a test strip as a specific binding analysis device in accordance with the present embodiment, and FIG. 7 is a schematic oblique view of a test strip in accordance with the present invention in which a plurality of units, shown in FIG. 6, are linked to one another. It is to be noted that the structure of the specific binding analysis device in accordance with the present embodiment is the same as that shown in FIG. 3.

As the present embodiment described is a case where the analyte is hCG, each of the first specific binding substance and the second specific binding substance is the monoclonal antibody to hCG, which is capable of participating in a sandwich reaction with hCG, and colloidal gold is used as the labeling material for direct labeling.

A solution of an anti-hCG monoclonal antibody, as the first specific binding substance, labeled with colloidal gold is sealed into the space forming zone 2 disposed on the developing layer 1, and the concentration, as well as an amount, of the anti-hCG monoclonal antibody solution to be sealed can be adjusted without restraint.

Further, the anti-hCG monoclonal antibody as the second specific binding substance is immobilized in the detection zone 6 on the developing layer 1, and the first electrode 11 and the second electrode 12 are disposed on the upper face of the developing layer 1. Further, the analysis device is constituted by a light source 7, a light detector 8, an analyzer 9 and a start-of-measurement detector 10 as a means to recognize introduction of a sample into the test strip. The detection zone 6 is irradiated with light having a prescribed wavelength (e.g. 520 nm) from the light source 7, and the reflected light is detected with the light detector 8 and then analyzed with the analyzer 9, to measure an absorption in the detection zone 6. As the measurement wavelength selected may be one suitable for the sample or exhibition of color of the labeling material in the detection zone 6.

The sample containing hCG is introduced into the test strip for a test reagent. When brought into contact with the inlet 3, the sample flows into the space forming zone 2 due to capillarity phenomenon. With the introduction of the sample, the developing layer 1 in a dry state changes to be in a wet state, and a conductivity of the developing layer 1 thus changes. Using the first electrode 11 and the second electrode 12, a conductivity of the developing layer 1 in a dry state is previously measured prior to the sample introduction and the conductivity is monitored during the sample introduction so that the start-of-measurement detector 10 can detect the sample introduction from the change in conductivity of the developing layer 1. Once the sample introduction is detected, the information on this detection is sent from the start-of-measurement detector 10 to the analyzer 9, which starts calculation of the measurement time on the assumption that the time at which the sample introduction is detected is zero.

The analyzer 9 has the function of controlling the light source 7 and the light detector 8 so as to automatically measure the absorption in the detection zone 6 at a previously predetermined time. The sample having flown into the space forming zone 2 comes into contact and then gets mixed with the solution of the anti-hCG monoclonal antibody, as the first specific binding substance, labeled with colloidal gold having been sealed in the space forming zone 2 so that the anti-hCG monoclonal antibody bound to colloidal gold and hCG in the sample form a labeled antigen-antibody complexes. The sample temporarily retained in the space forming zone 2 is discharged out of the outlet 4, develops along the developing layer 1 and arrives at the detection zone 6, where the labeled antigen-antibody complexes are bound to the immobilized anti-hCG monoclonal antibody, and thereby the labeled anti-hCG monoclonal antibody is trapped in the detection zone 6 to exhibit color.

The absorption of the labeling material in the detection zone 6 reflects the amount of hCG in the sample. In the analysis example of the present invention, a sampling inspection is previously conducted at the time of preparation of the analysis devices where the absorptions in the detection zones 6 are measured using a standard substance, and further, with the use of the analysis device from the same lot, the absorptions in the detection zone 6 measured with respect to the respective hCG concentrations are plotted to give a calibration curve. Herewith, a plurality of calibration curves for concentration conversion are prepared during preparation of the analysis devices, and then, when using the analysis devices, measurements are conducted using the standard sample, and from the absorption in the detection zone 6 shown by the standard sample, a calibration curve for concentration conversion to be applied to each of the analysis devices is determined. With this calibration curve utilized, the hCG concentration in the sample introduced to the developing layer 1 can be calculated from the absorption in the detection zone 6.

Subsequently, a sample containing hCG intended to be quantitatively analyzed is introduced into a strip from the same lot for a test reagent which comprises a plurality of space forming zones 2. The total amount of the anti-hCG monoclonal antibodies sealed in the respective space forming zones 2 in the strip is constant, whereas the concentration as well as the amount of the anti-hCG monoclonal antibody solution sealed in each of the space forming zones 2 are different. When brought into contact with the inlet 3, the sample flows into the space forming zone 2 due to capillary phenomenon; however, the amounts of the sample flowing in are different among the respective space forming zones 2 because of the difference in amount of the solution of the anti-hCG monoclonal antibody, as the first specific binding substance, labeled with colloidal gold, which is sealed in each of the space forming zones 2. Since the total amount of the anti-hCG monoclonal antibodies sealed in the respective space forming zones 2 is constant, the introduction of the sample into the test strip comprising a plurality of space forming zones 2 allows simultaneous observation of the specific binding reactions of the automatically diluted sample.

In the present invention, it is possible to make the sample automatically diluted in phases by previously adjusting the amount of the solution sealed in the space forming zone 2, and it is also possible to confirm as to whether there exists the concentration range where the prozone phenomenon is occurring, by observing and comparing the absorptions of the sample, diluted in phases, in the detection zone 6. Further, the hCG concentration in the sample introduced to the developing layer 1 can be calculated from the absorption of the sample, diluted to have a concentration not influenced by the prozone phenomenon, in the detection zone 6, with the use of the calibration curve. In such a case, the product of the dilution ratio and the analyte concentration obtained using the calibration curve is the analyte concentration in the sample.

### Embodiment 4 (Affinity)

A strip for a test reagent in the present embodiment is the same as in above Embodiment 2, except that the strip is constituted by the developing layers 1 with different affinities of the anti-hCG monoclonal antibodies as the second specific binding substances immobilized in the respective detection zones 6.

When the sample containing hCG is introduced into the strip, the absorption of the labeling material in the detection zone 6 reflects the amount of hCG in the sample. In the analysis example of the present invention, anti-hCG monoclonal antibodies each having a different affinity are prepared and immobilized in the detection zones 6 as the second specific binding substance, whereby the absorption in the detection zone 6 is different on each of the developing layers 1. Accordingly, the introduction of the sample into the test strip allows automatic and simultaneous observation of the antigen-antibody reactions having different signal intensities.

According to the present embodiment, it is possible to adjust, on each of the developing layers 1, the strength of the interaction between the analyte and the specific binding substance which are capable of participating in the specific binding reaction by previously adjusting the affinity of the anti-hCG monoclonal antibody as the second specific binding substance immobilized in the detection zone 6 on each of the developing layers 1, and it is also possible to calculate the hCG concentration in the sample introduced into the test strip by observing and comparing the results of the specific binding reactions with different strengths of the interactions between the analyte and the specific binding substance, and by utilizing the calibration curve, from the absorption of the labeling material in the detection zone 6 on the developing layer 1, where the antibody having the affinity not influenced by the prozone phenomenon is immobilized.

### Embodiment 5 (Particle size)

In the present embodiment, the same strip for a test reagent as in above Embodiment 3 is used, except that solutions of the anti-hCG monoclonal antibody, labeled with colloidal gold with different particle sizes, are adjusted to have a fixed absorption with a prescribed wavelength (e.g. 520 nm), which is then sealed in the same amount into each of the space forming zones 2 to constitute the strip.

A sample containing hCG intended to be quantitatively analyzed is introduced into a strip from the same lot for a test reagent which comprises a plurality of space forming zones 2. When colloidal gold as the labeling material have different particle sizes, the number of the labeling materials and the number of the antibodies, to be sealed, are different among the respective space forming zones 2. When the sample is introduced into the strip, reactions between the antigen and the antibody in different composition ratios are observed on the respective developing layers 1 due to steric hindrance which is caused by the particle size of colloidal gold and the difference in number of the labeling materials or the difference in number of the antibodies. Accordingly, the introduction of the sample into the strip comprising a plurality of space forming zones 2 allows simultaneous observation of the specific binding reactions with different signal intensities in the detection zones 6.

In the present embodiment, it is possible to automatically adjust, on each of the developing layers 1, the composition ratio of the analyte and the specific binding substance which are capable of participating in the specific binding reaction by utilizing the characteristic of the labeling material, and it is also possible to calculate the hCG concentration in the sample introduced into the test strip by observing and comparing the results of the specific binding reactions between the analyte and the specific binding substance in different composition ratios, and by utilizing the calibration curve, from the absorption of the labeling material in the detection zone 6 on the developing layer 1 with the composition ratio not influenced by the prozone phenomenon.

In the present embodiment, the corresponding relationship between the analyte concentration and the signal intensity in the detection zone 6 is previously assayed by the use of a standard substance with a known concentration, and the characteristic of the labeling material is utilized to adjust the concentration of the analyte, at which the prozone phenomenon occurs, to be different on each of the developing layers 1. It should be noted that the specific binding substance solution to be sealed into each of the space forming zones 2 is not necessarily in a fixed amount. Further, the method to be used for adjusting the amount of the specific binding substance solution is not limited to the method using the absorptions. Moreover, the characteristic of the labeling material is not limited to the particle size thereof, but various characteristics of the labeling material can be applied as necessary, according to each of labeling material agents.

### Embodiment 6 (Scanning)

In the present embodiment, the sample containing hCG is introduced into the strip to make the detection zone 6 exhibit color in the same manner as in Embodiment 3. The light source 7 and the light detector 8 are then scanned in the direction along the detection zone 6 so as to successively measure the signal intensities in the detection zones 6. It should be noted that the same effect can also be obtained by immobilizing the light source 7 and the light detector 8 and scanning the strip in a certain direction. Since the developing layers 1 are disposed in parallel while providing a gap between each of the layers, the signal intensities can be distinguishd among the respective developing layers 1 when the signal intensities are successively measured in a plurality of detection zones 6.

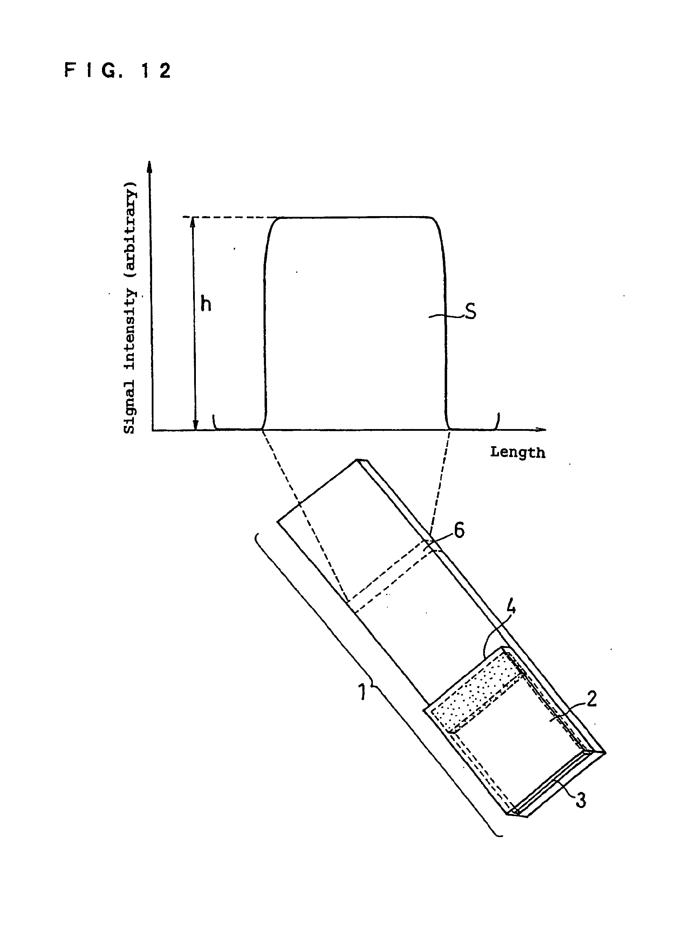

FIG. 12 is a schematic diagram of a curve drawn after analyzing the distribution status of the signal intensity in the detection zone 6 in the direction of the width of a developing layer 1. This curve was drawn after irradiating the upper face of the developing layer 1 with light from the light source 7 while scanning the developing layer 1 or the light source 7 and the light detector 8, and then analyzing, with the analyzer 9, reflected light detected with the light detector 8. In the diagram of this curve, the ordinate and the abscissa express the signal intensity and position in the detection zone region, respectively. It is to be noted that, in the present embodiment, the strip, or the light source 7 and the light detector 8, are scanned in the direction along the detection zone 6 to successively measure the reflected light in the detection zone 6 so that the signal intensity is obtained. The measurement is conducted, considering the height "h" of the curve as the signal intensity based on the specific binding reaction. Herein, the area "S" of the curve can be considered as the signal intensity based on the specific binding reaction. The absorption of the labeling material in the detection zone 6 reflects the amount of hCG in the sample.

In the analysis example of the present embodiment, a sampling inspection is previously conducted at the time of preparation of the analysis devices where the absorptions in the detection zones 6 are measured using a standard substance, and further, with the use of the analysis device from the same lot, the absorptions in the detection zone 6 measured with respect to the respective hCG concentrations are plotted to give a calibration curve. Herewith, a plurality of calibration curves for concentration conversion are prepared during preparation of the analysis devices, and then, when using the analysis devices, measurements are conducted using the standard sample, and from the absorption in the detection zone 6 shown by the standard sample, a calibration curve for concentration conversion to be applied to each of the analysis devices is determined. With this calibration curve utilized, the hCG concentration in the sample introduced to the developing layer 1 can be calculated from the absorption in the detection zone 6.

### Embodiment 7 (Scanning + affinity)

In the present embodiment, the sample containing hCG is introduced into the strip to make the detection zone 6 exhibit color in the same manner as in Embodiment 4. The light source 7 and the light detector 8 are then scanned in the direction along the detection zone 6 so as to successively measure the signal intensity in the detection zone 6. It should be noted that the same effect can also be obtained by immobilizing the light source 7 and the light detector 8 and scanning the strip in a certain direction. Since the developing layers 1 are disposed in parallel while providing a gap between each of the layers, the signal intensities can be distinguishd among the respective developing layers 1 when the signal intensities are successively measured in a plurality of detection zones 6.

The absorption in the detection zone 6 reflects the amount of the hCG in the sample. In the analysis example of the present invention, anti-hCG monoclonal antibodies each having a different affinity are prepared and immobilized in the detection zones 6 as the second specific binding substance, whereby the absorption in the detection zone 6 is different on each of the developing layers 1. Accordingly, the introduction of the sample into the test strip allows automatic and simultaneous observation of the antigen-antibody reactions having different signal intensities.

In the present embodiment, it is possible to adjust, on each of the developing layers 1, the strength of the interaction between the analyte and the specific binding substance which are capable of participating in the specific binding reaction by previously adjusting the affinity of the anti-hCG monoclonal antibody as the second specific binding substance immobilized in the detection zone 6 on each of the developing layers 1, and it is also possible to calculate the hCG concentration in the sample introduced into the test strip by observing and comparing the results of the specific binding reactions with different strengths of the interactions between the analyte and the specific binding substance, and by utilizing the calibration curve, from the absorption of the labeling material in the detection zone 6 on the developing layer 1, where the antibody having the affinity not influenced by the prozone phenomenon is immobilized.

In the present embodiment, the corresponding relationship between the analyte concentration and the signal intensity in the detection zone 6 is previously assayed by the use of a standard substance with a known concentration, and the characteristic of the labeling material is utilized to adjust the concentration of the analyte, at which the prozone phenomenon occurs, to be different on each of the developing layers 1. It should be noted that, in the embodiments of the present invention, the case is described where the difference in affinity of the monoclonal antibody is utilized as being easy to control; however, the characteristic of the specific binding substance is not limited to the affinity.

### Embodiment 8 (Scanning + particle size)

In the present embodiment, a sample containing hCG intended to be quantitatively analyzed is introduced into a strip for a test reagent which comprises a plurality of space forming zones 2, in the same manner as in Embodiment 6.
Herein, solutions of the anti-hCG monoclonal antibody, labeled with colloidal gold with different particle sizes, are adjusted to have a fixed absorption with a prescribed wavelength (e.g. 520 nm), which is then sealed in the same amount into each of the space forming zones 2 to constitute the strip. Thereat, when colloidal gold, as the labeling material, have different particle sizes, the number of the labeling materials and the number of the antibodies, to be sealed, are different among the respective space forming zones 2. When the sample is introduced into the strip, reactions between the antigen and the antibody in different composition ratios are observed on the respective developing layers 1 due to steric hindrance which is caused by the particle size of colloidal gold and the difference in number of the labeling materials or the difference in number of the antibodies. Accordingly, the introduction of the sample into the test strip comprising a plurality of space forming zones 2 allows simultaneous observation of the specific binding reactions with different signal intensities in the detection zones 6.

In the present embodiment, it is possible to automatically adjust, on each of the developing layers 1, the composition ratio of the analyte and the specific binding substance which are capable of participating in the specific binding reaction by utilizing the characteristic of the labeling material, and it is also possible to calculate the hCG concentration in the sample introduced into the test strip by observing and comparing the results of the specific binding reactions between the analyte and the specific binding substance in different composition ratios, and by utilizing the calibration curve, from the absorption of the labeling material in the detection zone 6 on the developing layer 1 with the composition ratio not influenced by the prozone phenomenon.

In the present embodiment, the corresponding relationship between the analyte concentration and the signal intensity in the detection zone 6 is previously assayed by the use of a standard substance with a known concentration, and the characteristic of the labeling material is utilized to adjust the concentration of the analyte, at which the prozone phenomenon occurs, to be different on each of the developing layers 1. It should be noted that the specific binding substance solution to be sealed into each of the space forming zones 2 is not necessarily in a fixed amount. Further, the method to be used for adjusting the amount of the specific binding substance solution is not limited to the method using the absorptions. Moreover, the characteristic of the labeling material is not limited to the particle size thereof, but various characteristics of the labeling material can be applied as necessary, according to each of labeling material agents.

In the following, the present invention is described more specifically by means of examples; however, the present invention is not limited thereto.

### Example 1

The present example was performed in line with aforesaid Embodiment 1 using the specific binding analysis device in accordance with FIGS. 1 to 3. A strip comprising five units shown in FIG. 2 was used. A monoclonal antibody to hCG capable of participating in a sandwich reaction with hCG was used as a first specific binding substance and a second specific binding substance, and colloidal gold was used as a labeling material to directly label the first specific binding substance. Urine added with a known amount of hCG was used as a sample, and when the detection zone 6 was irradiated with light having a wavelength of 520 nm, a signal intensity obtained in the light detector 8 was measured, to quantitatively measure the hCG concentration in the sample. As the sample used was urine with an hCG concentration of 50 IU/L, 100 IU/L, 250 IU/L, 500 IU/L or 1,000 IU/L. These samples were introduced from each inlet 3 of the five units.

Further, the introduction of the sample was detected with the start-of-measurement detector 10 from the change in conductivity of the developing layer 1, and the measurement time was determined on the assumption that the start-of-detection time was zero. The space forming zone 2 with a volume of 50 µl was used. After being brought into contact with the inlet 3, the sample filled the space forming zone 2 within five seconds, and finished developing through the test strip in three minutes. Hence the rate of introducing the sample was sufficiently faster than the rate of discharging the sample. The volume of the space forming zone 2 is not particularly limited and is set up accordingly to the sample, analysis device or the like.

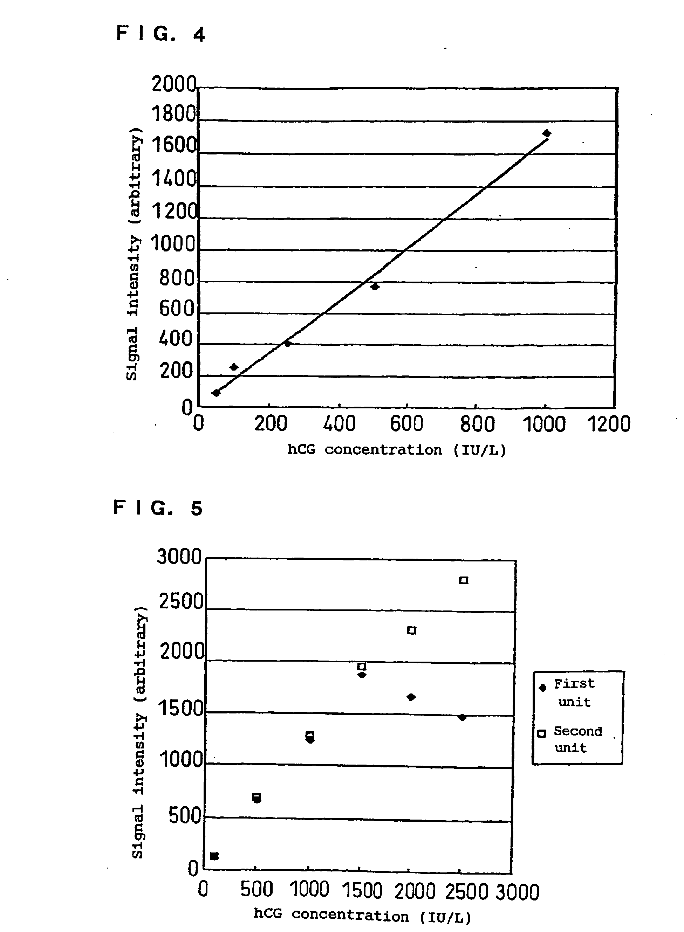

FIG. 4 is a graph of the signal intensities measured in the detection zone 6 at 520 nm, five minutes after the introduction of the sample into the test strip. In terms of each of the hCG concentrations, the signal intensity increases dependently on the concentration, indicating that they are in the linear relationship. Although not shown in the drawing, when a sample, with the hCG concentration being substantially zero as indicated by the detector, was introduced into the test strip, the signal intensity in the detection zone 6 at 520 nm was zero. In such a manner, the use of FIG. 4 as the calibration curve allows measurement of the hCG concentration in the sample with a favorable quantitative property.

### Example 2

The present example was performed in line with aforesaid Embodiment 2 using the specific binding analysis device in accordance with FIGS. 1 to 3. A test strip comprising two units was used here. The monoclonal antibody to hCG capable of participating in a sandwich reaction with hCG was used as the first specific binding substance and the second specific binding substance, and colloidal gold was used as the labeling material to directly label the first specific binding substance. Urine added with a known amount of hCG was used as the sample, and a signal intensity in the detection zone 6 at 520 nm was measured. As the sample used was urine with an hCG concentration of 100 IU/L, 500 IU/L, 1,000 IU/L, 1,500 IU/L, 2,000 IU/L or 2,500 IU/L.

Further, the introduction of the sample was detected with the start-of-measurement detector 10 from the change in conductivity of the developing layer 1, and the measurement time was determined on the assumption that the start-of-detection time was zero. The amount of the first specific binding substance, directly labeled by the use of colloidal gold, retained in the retention zone 5 in the first unit was adjusted so as to be one tenth the amount retained in the second unit. That is, five single units are in a linked state in FIG. 2, whereas the strip with the configuration of comprising two units (the first unit and the second unit) linked to one another was used in the present example. Namely, samples with the aforesaid six kinds of hCG concentrations were introduced into both the first unit and the second unit.

FIG. 5 is a graph showing the relationships between the hCG concentrations and the signal intensities in the detection zones 6 at 520 nm, measured five minutes after detection of the sample introduction, in the first unit and the second unit.

The signal intensity measured five minutes after the detection of the sample introduction in the first unit increases with an increase in hCG concentration when the concentration is low (hCG concentration = 100 IU/L, 500 IU/L, 1,000 IU/L, 1,500 IU/L); however, on the contrary, it decreases with an increase in hCG concentration when the concentration is as high as not less than 2,000 IU/L, whereby it is found that there may exist a plurality of hCG concentrations which correspond to one signal intensity, depending on the concentration range.

In this case, that is, the presence of excess hCG in the sample causes competition between hCG not specifically bound to the first specific binding substance labeled with colloidal gold and hCG specifically bound to the first specific binding substance labeled with colloidal gold, in the specific binding reaction with the second specific binding substance in the detection zone 6, resulting in a decrease in amount of the first specific binding substance labeled with colloidal gold, which is bound in the detection zone 6, and a decrease in signal intensity, to which colloidal gold contributes, and hence the signal intensity detected in the detection zone 6 does not reflect the amount of hCG in the sample.

In the second unit, in terms of each of the hCG concentrations, the signal intensity increases dependently on the concentration, indicating that they are in the linear relationship. Although not shown in the drawing, when a sample, with the hCG concentration being substantially zero as indicated by the detector, was introduced into the second unit, the signal intensity in the detection zone 6 at 520 nm was zero. This means that in the second unit, by increasing the amount of the anti-hCG monoclonal antibody, as the first specific binding substance labeled with colloidal gold, retained in the retention zone 5, the amount of hCG not specifically bound to the first specific binding substance, labeled with colloidal gold, which had caused the prozone phenomenon observed in the first unit, decreased, to avoid the influence of the prozone phenomenon.

As thus described, it is possible to calculate the hCG concentration in the sample introduced into the test strip by previously adjusting the amount of the anti-hCG monoclonal antibody, as the first specific binding substance, labeled with colloidal gold, retained in the retention zone 5 in each of the units to automatically adjust, in each of the units, the composition ratio of the analyte and the specific binding substance which are capable of participating in the specific binding reaction, and by utilizing the calibration curve, from the absorption of the labeling material in the detection zone 6 on the developing layer 1 with the composition ratio not influenced by the prozone phenomenon. Table 1 showed the signal intensity ratios in the detection zones 6 of the first unit and the second unit, determined from FIG. 5.

**Table 1**

| hCG concentration (IU/L) | Signal intensity ratio |
|---|---|
| | (first unit/second unit) |
| 100 | 0.962 |
| 500 | 0.964 |
| 1000 | 0.969 |
| 1500 | 0.959 |
| 2000 | 0.724 |
| 2500 | 0.529 |

The signal intensity ratios are almost constant when the hCG concentration is low (hCG concentration = 100 IU/L, 500 IU/L, 1,000 IU/L, 1,500 IU/L), whereas the signal intensity ratios decrease with an increase in the hCG concentration when the concentration is as high as not less than 2,000 IU/L. Whether the prozone phenomenon has occurred can thus be confirmed by introducing the sample into the test strip where the amount of the anti-hCG monoclonal antibody, as the first specific binding substance, labeled with colloidal gold, retained in the retention zone 5 in each of the units, has been adjusted, and then calculating the ratios of the signal intensities in the respective detection zones 6 followed by observation and comparison.

### Example 3

The present example was performed in line with aforesaid Embodiment 3 using the specific binding analysis device in accordance with FIGS. 3, 6 and 7. The number of the units in FIG. 7 was five whereas the number of the units here was six. The monoclonal antibody to hCG capable of participating in a sandwich reaction with hCG was used as the first specific binding substance and the second specific binding substance, and colloidal gold was used as the labeling material to directly label the first specific binding substance. Herein, an equal amount of a solution of an anti-hCG monoclonal antibody, as the first specific binding substance, was sealed into each of the space forming zones 2. Urine added with a known amount of hCG was used as the sample, and a signal intensity in the detection zone 6 at 520 nm was measured.

As the sample used here was urine with an hCG concentration of 100 IU/L, 500 IU/L, 1,000 IU/L, 1,500 IU/L, 2,000 IU/L or 2,500 IU/L. Such samples were introduced from each inlet 3 of the six units. Further, the introduction of the sample was detected with the start-of-measurement detector 10 from the change in conductivity of the developing layer 1, and the measurement time was determined on the assumption that the start-of-detection time was zero.

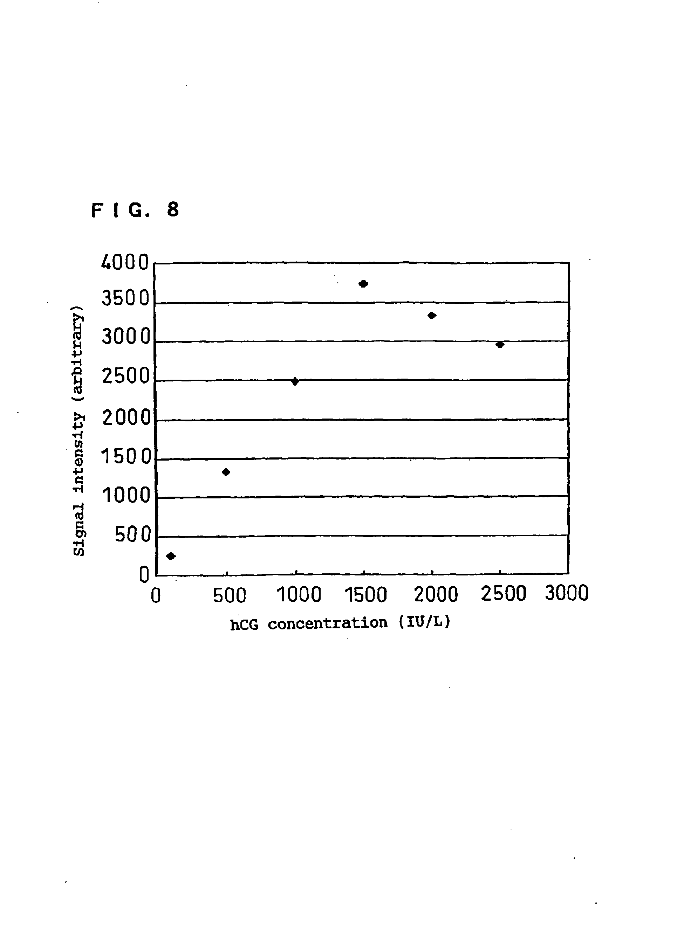

FIG. 8 is a graph showing the relationships between the hCG concentrations and the signal intensities in the detection zones 6 at 520 nm, measured five minutes after the detection of the sample introduction, in the strip comprising the six units. The signal intensity measured five minutes after the detection of the sample introduction increases with an increase in hCG concentration when the concentration is low (hCG concentration = 100 IU/L, 500 IU/L, 1,000 IU/L, 1,500 IU/L); however, on the contrary, it decreases with an increase in hCG concentration when the concentration is as high as not less than 2,000 IU/L, whereby it is found that there may exist a plurality of hCG concentrations which correspond to one signal intensity, depending on the concentration range.

In this case, that is, the presence of excess hCG in the sample causes competition between hCG not specifically bound to the first specific binding substance labeled with colloidal gold and hCG specifically bound to the first specific binding substance labeled with colloidal gold, in the specific binding reaction with the second specific binding substance in the detection zone 6, resulting in a decrease in amount of the first specific binding substance labeled with colloidal gold, which is bound in the detection zone 6, and a decrease in signal intensity, to which colloidal gold contributes, and hence the signal intensity detected in the detection zone 6 does not reflect the amount of hCG in the sample.

Next, urine with an hCG concentration of 2,500 IU/L was introduced into each of the units of the test strip comprising five space forming zones 2. The test strip was used where the total amount of the anti-hCG monoclonal antibodies sealed in the respective space forming zones 2 was constant, but the concentration as well as the amount of the anti-hCG monoclonal antibody solution sealed in each of the space forming zones 2 were made different; to be more specific, the anti-hCG monoclonal antibody solutions were adjusted such that the final hCG concentrations of the samples to be developed along the developing layers 1 were, respectively, 100 IU/L, 500 IU/L, 1,000 IU/L, 1,500 IU/L and 2,000 IU/L, which were then sealed into the space forming zones 2.

In the present invention, when the sample was brought into contact with the inlet 3, a certain amount of the sample flowed in due to capillary phenomenon; however, the amount of the sample flowing in was different in each of the space forming zones 2 because of the difference in amount of the solution of the anti-hCG monoclonal antibody, as the first specific binding substance, labeled with colloidal gold, which was sealed in each of the space forming zones 2. Since the total amount of the anti-hCG monoclonal antibodies sealed in the respective space forming zones 2 was constant, the introduction of the sample into the test strip comprising a plurality of space forming zones 2 allowed simultaneous observation of the specific binding reactions of the automatically diluted sample.

As a result of measuring the signal intensity in the detection zone 6 at 520 nm, five minutes after detection of the sample introduction, accuracy of the sample dilution was confirmed. Further, the signal intensity increased with an increase in hCG concentration when the concentration was low (hCG concentration = 100 IU/L, 500 IU/L, 1,000 IU/L, 1,500 IU/L); however, it decreased with an increase in hCG concentration when the concentration was 2,000 IU/L.

In the present invention, as thus described, it is possible to automatically dilute the sample in phases by previously adjusting the amount of the solution sealed in the space forming zone 2.

### Example 4

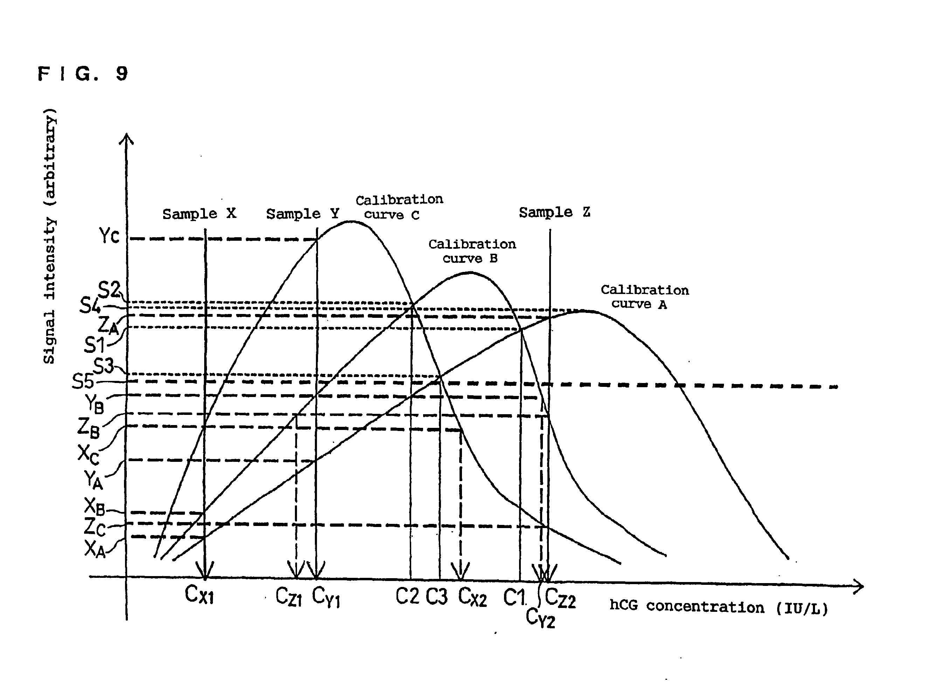

Next, a specific binding analysis device with a configuration of linking three single units was used. In this case, the space forming zone 2 was disposed in each of Unit A, Unit B and Unit C. FIG. 9 showed a graph of overlapped calibration curves each obtained by measuring the signal intensity in the detection zone 6 by the use of a sample containing hCG with a known concentration as the sample in each of Unit A, Unit B and Unit C. The calibration curve A, the calibration curve B and the calibration curve C were corresponded to Unit A, Unit B and Unit C, respectively.

Unit A, Unit B and Unit C were used under the same conditions except for the amount of the solution of the anti-hCG monoclonal antibody labeled with colloidal gold, which was sealed in each of the space forming zones 2. Although the total amount of the anti-hCG monoclonal antibodies sealed in the respective space forming zones 2 in Unit A. Unit B and Unit C was constant, the amount of the antibody solution sealed in each of the space forming zones 2 was made smaller in the order of Unit A, Unit B and Unit C, i.e., the amount of Unit A > the amount of Unit B > the amount of Unit C.

The inclination of the calibration curve in the straight line region where the prozone phenomenon was not occurring was steeper in the order of Unit C, Unit B and Unit A since, upon introduction of the sample containing hCG into the space forming zone 2, the larger the amount of the sealed antibody solution, the more sample was diluted. As for each of the calibration curves A, B and C, when the hCG concentration was low, the signal intensity increased with an increase in concentration; when the hCG concentration was high, the prozone phenomenon was observed.

The intersection of the calibration curves A and B was (C1, S1), the intersection of the calibration curves B and C was (C2, S2) and the intersection of the calibration curves A and C was (C3, S3), and a threshold S5 was set, which was less than the minimum signal intensity S3 among the values of signal intensities of the respective calibration curves and also less than the minimum signal intensity S4 among the maximum values of the signal intensities of the respective calibration curves. The signal intensities in the detection zones 6 when Sample X, Sample Y and Sample Z, each containing hCG, were developed in the strips were (X_{A}, X_{B}, X_{C}), (Y_{A}, Y_{B}, Y_{C}), (Z_{A}, Z_{B}, Z_{C}) in the order of Unit A, Unit B and Unit C.

With Sample X, the magnitudes of the signal intensities were represented by X_{A}<X_{B}<X_{C} and X_{C}<S5. In the present invention, the hCG concentration in the sample could be measured by using the signal intensity X_{C} shown in Unit C which showed the largest signal intensity among the reaction fields to show signal intensities not more than the threshold S5 (Units A, B, C). When the hCG concentration in the sample was less than the minimum value C2 of the hCG concentration at the intersection generated when overlapping the calibration curves A, B and C, as comparing the signal intensities on the reaction fields, where the sample was introduced and the specific binding reaction was conducted, the steeper the inclination of the calibration curve in the straight line region where the prozone phenomenon was not occurring, the larger the signal intensity shown by hCG in the sample.

In such a case where the magnitude relationship of the signal intensities shown by the analyte in the sample on the reaction fields, where the sample was introduced and the specific binding reaction was conducted, corresponded to the magnitude relationship of the inclinations of the calibration curves in the straight line regions where the prozone phenomenon was not occurring, the smaller concentration Cₓ₁ between Cₓ₁ and Cₓ₂ corresponding to the signal intensity X_{C} obtained from the calibration curve C was the hCG concentration in the sample. In this case, the product of the dilution ratio and the analyte concentration Cₓ₁ obtained using the calibration curve C was the analyte concentration in the sample.

With the sample Y, the magnitudes of the signal intensities were expressed by Y_{A}<Y_{B}<Y_{C} and Y_{B}<S5<Y_{C}. In the present invention, the hCG concentration in the sample could be measured by using the signal intensity Y_{B} shown in Unit B which showed the largest signal intensity among the reaction fields to show signal intensities not more than the threshold S5 (Units A, B). In such a case where the magnitude relationship of the signal intensities shown by the analyte in the sample on the reaction fields, where the sample was introduced and the specific binding reaction was conducted, corresponded to the magnitude relationship of the inclinations of the calibration curves in the straight line regions where the prozone phenomenon was not occurring, the smaller concentration C_{Y1} between C_{Y1} and C_{Y2} corresponding to the signal intensity Y_{B} obtained from the calibration curve B was the hCG concentration in the sample. In this case, the product of the dilution ratio and the analyte concentration C_{Y1} obtained from the calibration curve C was the analyte concentration in the sample. With the sample Z, the magnitudes of the signal intensities were expressed by Z_{C}<Z_{B}<Z_{A} and Z_{B}<S5<Z_{A}.

In such a manner, when the magnitude relationship of the signal intensities shown by the analyte in the sample on the reaction fields, where the sample was introduced and the specific binding reaction was conducted, did not correspond to the magnitude relationship of the inclinations of the calibration curves in the straight line regions where the prozone phenomenon was not occurring, namely, when the hCG concentration in the sample was not less than the minimum value C2 of the hCG concentration at the intersection generated when overlapping the calibration curves A, B and C, the specific binding analysis device in the present invention could consider the hCG concentration in the sample as being out of the scope of the qualitatively or quantitatively analysis. In this case, the sample Z was further diluted or a specific binding analysis device adaptable to a high hCG concentration was used so as to qualitatively or quantitatively analyze hCG in the sample.

As thus described, it is possible to qualitatively or quantitatively analyze the analyte in the sample without being influenced by the prozone phenomenon which has been a problem with the conventional specific binding reaction, by comparing the magnitude relationship of the absorptions of the samples, diluted in phases, in the detection zones 6 on the respective development layers 1 constituting the test strip, with the magnitude relationship of the inclinations of the calibration curves in the straight line regions where the prozone phenomenon is not occurring, and then analyzing the signal intensity on the reaction field to show the largest signal intensity among reaction fields where the specific binding reaction is conducted to show signal intensities not more than a threshold set up by utilizing the present invention. In this case, the product of the dilution ratio and the analyte concentration obtained from the calibration curve is the analyte concentration in the sample.

### Example 5 (Affinity)

The present example was performed in line with aforesaid Embodiment 4 using the specific binding analysis device in accordance with FIGS. 1 to 3. Hence the same operation was conducted as in Example 2, except that a strip comprising a first unit and a second unit was prepared and anti-hCG monoclonal antibodies having different affinities (10¹¹M⁻¹ and 10¹⁰M⁻¹) were immobilized, respectively, in the detection zones 6 of the first unit and the second unit.

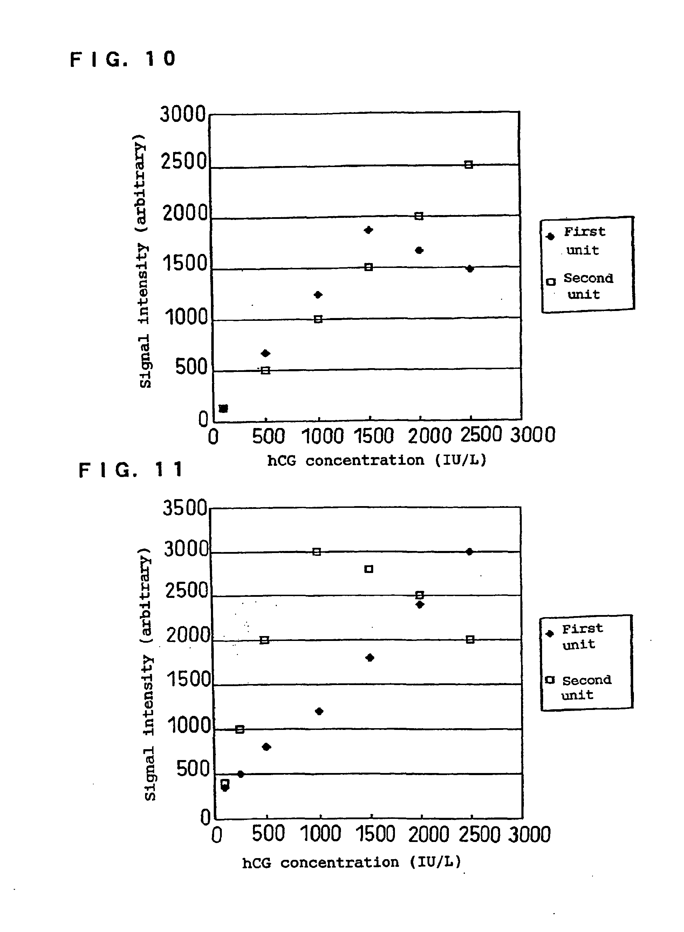

FIG. 10 is a graph showing the relationships between the hCG concentrations and the signal intensities in the respective detection zones 6 at 520 nm, measured five minutes after the detection of the sample introduction, in the first unit and the second unit.

The signal intensity in the first unit measured five minutes after the detection of the sample introduction increases with an increase in hCG concentration when the concentration is low (hCG concentration = 100 IU/L, 500 IU/L, 1,000 IU/L, 1,500 IU/L); however, on the contrary, it decreases with an increase in hCG concentration when the concentration is as high as not less than 2,000 IU/L, whereby it is found that there may exist a plurality of hCG concentrations which correspond to one signal intensity, depending on the concentration range.

Further, in the second unit, in terms of each of the hCG concentrations, the signal intensity increases dependently on the concentration, indicating that they are in the linear relationship. Although not shown in the drawing, when a sample, with the hCG concentration being substantially zero as indicated by the detector, was introduced into the strip, the signal intensity in the detection zone 6 at 520 nm was zero. This means that the use of the antibody with lower affinity in the second unit as compared to the antibody immobilized in the first unit weakened the strength of the interaction with the analyte, changing the balance between the analyte trapped due to the specific binding reaction in the detection zone 6 and the analyte washed off out of the detection zone 6 along with the flow of the sample so that the analyte concentration observed as the prozone phenomenon shifts to the higher concentration side than the developing layer 1, thereby resulting in avoidance of the influence of the prozone phenomenon in the second unit.

It is thereby found that the hCG concentration in the sample introduced into the test strip can be calculated by previously adjusting the affinity of the anti-hCG monoclonal antibody to be immobilized in the detection zone 6 in each of the units to adjust the strength of the interaction with the analyte and by utilizing the calibration curve, from the absorption of the labeling material in the detection zone 6 in the unit, where the antibody with an affinity not influenced by the prozone phenomenon is immobilized.

### Example 6

Next, a specific binding analysis device with a configuration of linking three single units in the same manner as in Example 4 was used and the same operation as in Example 5 was conducted. A graph was created by overlapping calibration curves each obtained by measuring the signal intensity in the detection zone 6, using a sample containing hCG with a know concentration as the sample in each of Unit A, Unit B and Unit C, which was the same graph as the one shown in FIG. 9.

Also in the present invention, therefore, it is possible to qualitatively or quantitatively analyze the analyte in the sample without being influenced by the prozone phenomenon which has been a problem with the conventional specific binding reaction, by comparing the magnitude relationship of the absorptions in the detection zones 6 in the respective units constituting the strip, with the magnitude relationship of the inclinations of the calibration curves in the straight line regions where the prozone is not occurring, and then analyzing a signal intensity on the reaction field to show the largest signal intensity among reaction fields where specific binding reaction is conducted to show signal intensities not more than a threshold set up by utilizing the present invention.

### Example 7 (Particle size)

The same operation as in Example 5 was conducted in line with aforesaid Embodiment 5 where the particle size of colloidal gold as the labeling material is changed.

It is to be noted that urine with an hCG concentration of 100 IU/L, 250 IU/L, 500 IU/L, 1,000 IU/L, 1,500 IU/L, 2,000 IU/L or 2,500 IU/L was used as the sample.

A strip comprising a first unit and a second unit was prepared and colloidal gold with particle sizes of 20 nm and 70 nm were used as the labeling materials for the anti-hCG monoclonal antibodies as the first specific binding substances in the respective units. The equal amount of the solution of the anti-hCG monoclonal antibody labeled with each of the colloidal gold, with the absorption at 520 nm made constant, was sealed into each of the space forming zones.

FIG. 11 is a graph showing the relationships between the hCG concentrations and the signal intensities in the detection zones 6 at 520 nm, measured five minutes after the detection of the sample introduction, in the first unit and the second unit. The signal intensity in the second unit measured five minutes after the detection of the sample introduction increases with an increase in hCG concentration when the concentration is low (hCG concentration = 100 IU/L, 250 IU/L, 500 IU/L, 1,000 IU/L). However, on the contrary, it decreases with an increase in hCG concentration when the concentration is as high as not less than 1,500 IU/L. That is to say, there may exist a plurality of hCG concentrations which correspond to one signal intensity, depending on the concentration range.

In this case, the presence of excess hCG in the sample causes competition between hCG not specifically bound to the first specific binding substance labeled with colloidal gold and hCG specifically bound to the first specific binding substance labeled with colloidal gold, in the specific binding reaction with the second specific binding substance in the detection zone 6. As a result, the amount of the first specific binding substance labeled with colloidal gold, which is bound in the detection zone 6, decreases and the signal intensity, to which colloidal gold contributes, decreases, and hence the signal intensity detected in the detection zone 6 does not reflect the amount of hCG in the sample.

In the first unit, in terms of each of the hCG concentrations, the signal intensity increases dependently on the concentration, indicating that the linear relationship has been formed therebetween. Although not shown in the drawing, when a sample, with the hCG concentration being substantially zero as indicated by the detector, was introduced into the test strip, the signal intensity in the detection zone 6 at 520 nm was zero. This means that in the first unit, by making the particle size of colloidal gold as the labeling material smaller, the number of the anti-hCG monoclonal antibodies as the first specific binding substances which were sealed in the space forming zone increased while the amount of hCG not specifically bound to the first specific binding substance labeled with colloidal gold, which had caused the prozone phenomenon observed in the second unit, decreased, to avoid the influence of the prozone phenomenon.

It is thereby found that the hCG concentration in the sample introduced into the strip can be calculated by previously adjusting the characteristic of the labeling material to adjust the number of the anti-hCG monoclonal antibodies as the first specific binding substances labeled with colloidal gold in the respective units and automatically adjust the composition ratio of the analyte and the specific binding substance which are capable of participating in the specific binding reaction, and by utilizing the calibration curve, from the absorption of the labeling material in the detection zone 6 in the unit with the composition ratio not influenced by the prozone phenomenon.

### Example 8 (Scanning)

In line with aforesaid Embodiment 6 using the specific binding analysis device in accordance with FIGS. 1 to 3, the monoclonal antibody to hCG capable of participating in a sandwich reaction with hCG was used as the first specific binding substance and the second specific binding substance, and colloidal gold was used as the labeling material to directly label the first specific binding substance. Urine added with a known amount of hCG was used as the sample, and a signal intensity in the detection zone at 520 nm was measured. As the sample used was urine with an hCG concentration of 100 IU/L, 250 IU/L, 500 IU/L or 1,000 IU/L. Further, the introduction of the sample was detected with the start-of-measurement detector 10 from the change in conductivity of the developing layer 1, and the measurement time was determined on the assumption that the start-of-detection time was zero.

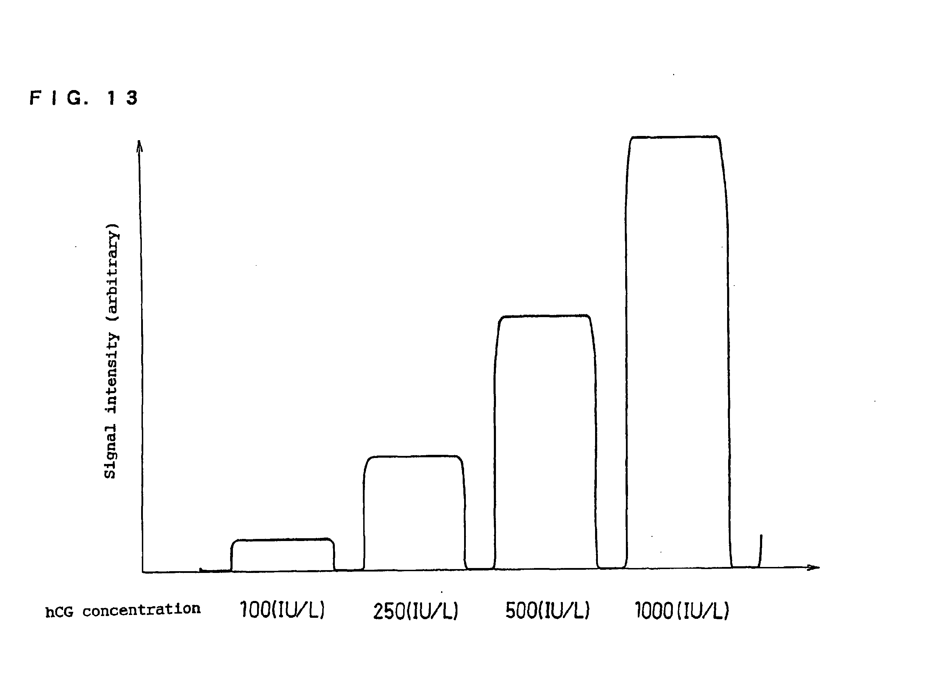

FIG. 13 represented the distribution status of the signal intensity in the detection zone 6 at 520 nm, measured five minutes after the detection of the sample introduction in a strip constituted by disposing four units on parallel. As evident from the drawing, since the developing layers 1 are disposed in parallel while providing a gap between each of the layers, the signal intensities in the respective units can be distinguishd when the signal intensities in a plurality of detection zones 6 are successively measured.

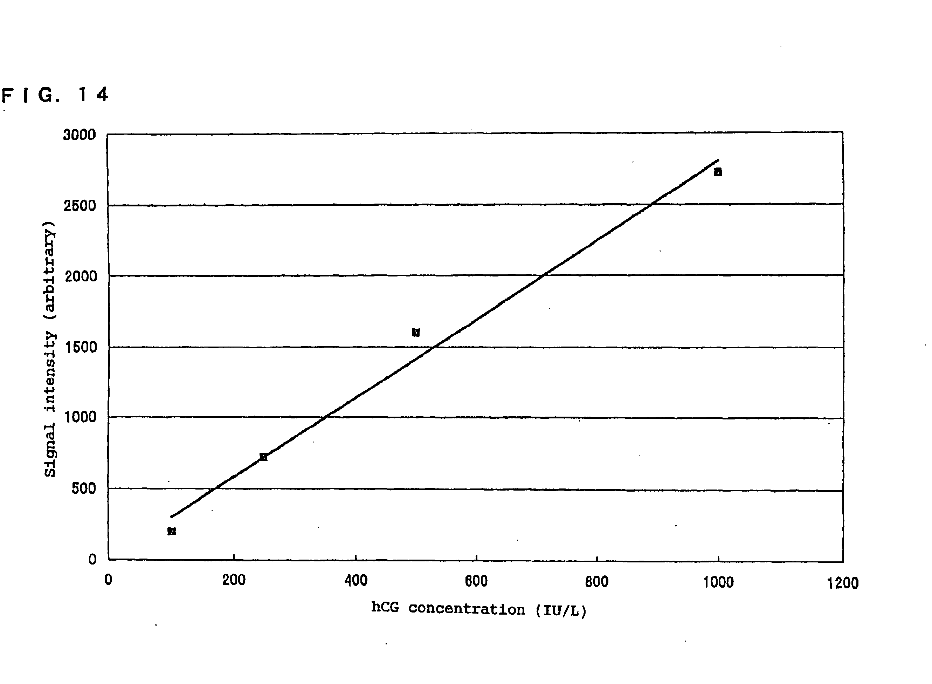

FIG. 14 is a graph showing the relationship between the hCG concentration and the signal intensity in the detection zone 6 at 520 nm, measured five minutes after the detection of the sample introduction, when it is assumed that the height of the curve in FIG. 13 was the signal intensity in the detection zone of each of the units. In terms of each of the hCG concentration, the signal intensity increases dependently on the concentration, indicating that the linear relationship has been formed therebetween. Although not shown in the drawing, when a sample, with the hCG concentration being substantially zero as indicated by the detector, was introduced into the test strip, the signal intensity in the detection zone 6 at 520 nm was zero.

### Example 9 (Scanning + Affinity)

In line with aforesaid Embodiment 7 using the specific binding analysis device in accordance with FIGS. 1 to 3, the monoclonal antibody to hCG capable of participating in a sandwich reaction with hCG was used as the first specific binding substance and the second specific binding substance, and colloidal gold was used as the labeling material to directly label the first specific binding substance. Urine added with a known amount of hCG was used as the sample, and a signal intensity in the detection zone at 520 nm was measured. As the sample used was urine with an hCG concentration of 100 IU/L, 500 IU/L, 1,000 IU/L, 1,500 IU/L, 2,000 IU/L or 2,500 IU/L.

Further, the introduction of the sample was detected with the start-of-measurement detector 10 from the change in conductivity of the developing layer 1, and the measurement time was determined on the assumption that the start-of-detection time was zero. A strip comprising a first unit and a second unit was prepared and anti-hCG monoclonal antibodies having different affinities (10¹¹M⁻¹ and 10¹⁰M⁻¹) were immobilized, respectively, in the detection zones 6 of the first unit and the second unit.

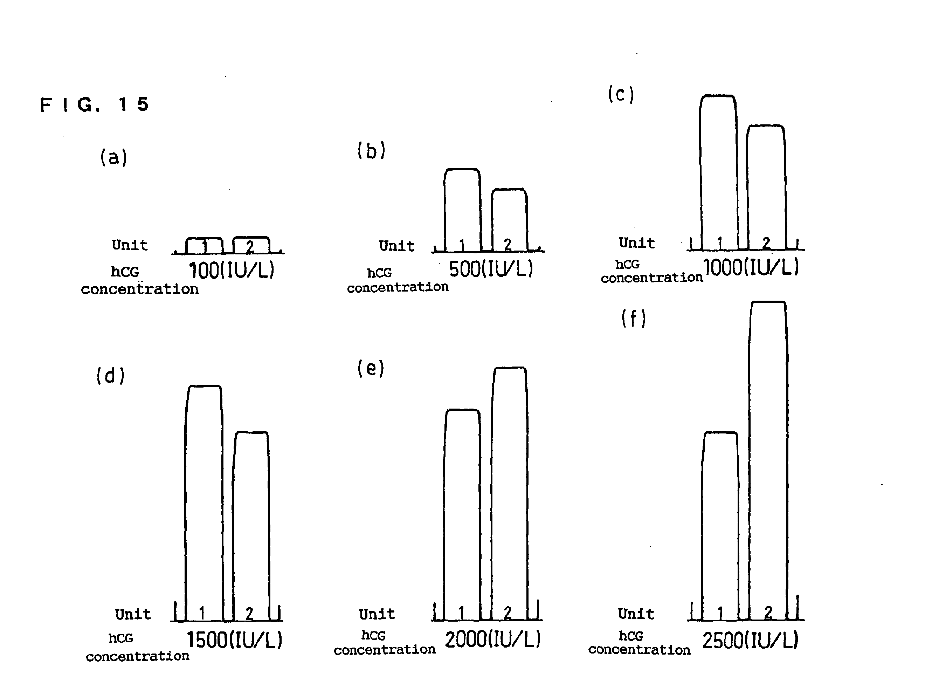

(a) to (f) in FIG. 15 represented the distribution statuses of the signal intensities in the detection zones 6 at 520 nm, measured five minutes after detecting the introduction of the samples with different hCG concentrations. As obvious from the drawing, since the first unit is disposed in parallel with the second unit while providing a gap therebetween, the signal intensities in the respective units can be distinguishd when the signal intensities in a plurality of detection zones 6 are successively measured.

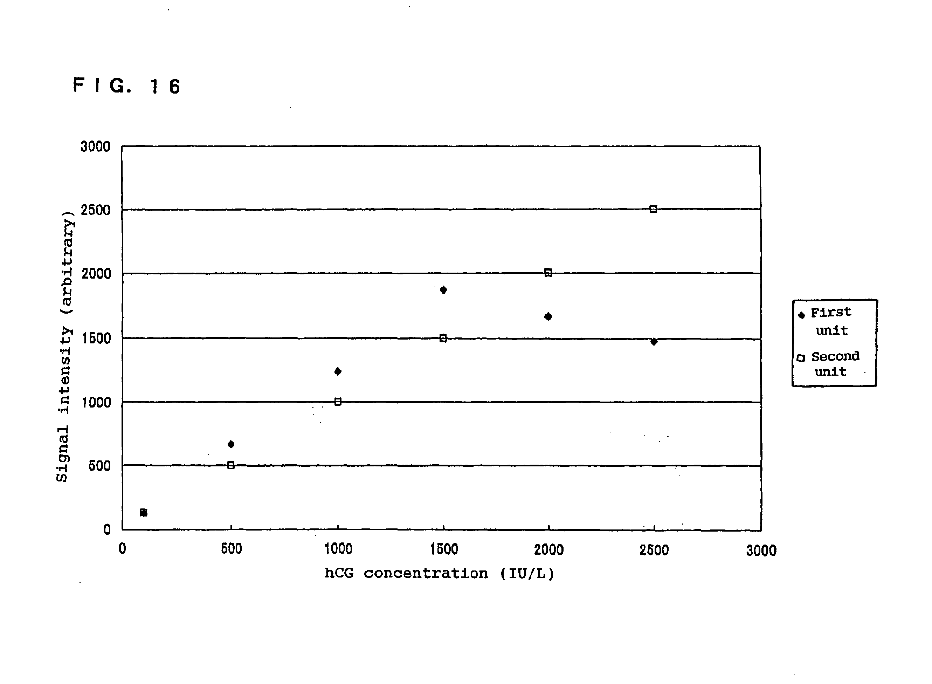

FIG. 16 is a graph showing the relationships between the hCG concentrations and the signal intensities in the detection zones 6 at 520 nm, measured five minutes after the detection of the sample introduction, when it is assumed that the height of the curve in FIG. 15 was the signal intensity in the detection zone of each of the units.

The signal intensity in the first unit measured five minutes after the detection of the sample introduction increases with an increase in hCG concentration when the concentration is low (hCG concentration = 100 IU/L, 500 IU/L, 1,000 IU/L, 1,500 IU/L); however, on the contrary, it decreases with an increase in hCG concentration when the concentration is as high as not less than 2,000 IU/L, whereby it is found that there may exist a plurality of hCG concentrations which correspond to one signal intensity, depending on the concentration range.

In this case, namely, the presence of excess hCG in the sample causes competition between hCG not specifically bound to the first specific binding substance labeled with colloidal gold and hCG specifically bound to the first specific binding substance labeled with colloidal gold, in the specific binding reaction with the second specific binding substance in the detection zone 6, resulting in a decrease in amount of the first specific binding substance labeled with colloidal gold, which is bound in the detection zone 6, and a decrease in signal intensity, to which colloidal gold contributes, and hence the signal intensity detected in the detection zone 6 does not reflect the amount of hCG in the sample.

In the second unit, in terms of each of the hCG concentrations, the signal intensity increases dependently on the concentration, indicating that they are in the linear relationship. Although not shown in the drawing, when a sample, with the hCG concentration being substantially zero as indicated by the detector, was introduced into the test strip, the signal intensity in the detection zone 6 at 520 nm was zero. The use of the antibody with lower affinity in the second unit as compared to the antibody immobilized in the first unit weakened the strength of the interaction with the analyte, thereby changing the balance between the analyte trapped due to the specific binding reaction in the detection zone 6 and the analyte washed off out of the detection zone along with the flow of the sample. The analyte concentration observed as the prozone phenomenon then shifts to the higher concentration side than the developing layer 1, meaning that, as a result, the influence of the prozone phenomenon is avoided in the second unit.

It is thereby found that the hCG concentration in the sample introduced into the test strip can be calculated by previously adjusting the affinity of the anti-hCG monoclonal antibody to be mobilized in the detection zone 6 in each of the units to adjust the strength of the interaction with the analyte, and by utilizing the calibration curve, from the absorption of the labeling material in the detection zone in the unit, where the antibody with the affinity not influenced by the prozone phenomenon is immobilized.

### Example 10 (Scanning + Particle size)

In line with aforesaid Embodiment 8 using the specific binding analysis device in accordance with FIGS. 1 to 3, the monoclonal antibody to hCG capable of participating in a sandwich reaction with hCG was used as the first specific binding substance and the second specific binding substance, and colloidal gold was used as the labeling material to directly label the first specific binding substance. Urine added with a known amount of hCG was used as the sample, and a signal intensity in the detection zone at 520 nm was measured. As the sample used was urine with an hCG concentration of 100 IU/L, 250 IU/L, 500 IU/L, 1,000 IU/L, 1,500 IU/L, 2,000 IU/L or 2,500 IU/L.

Further, the introduction of the sample was detected with the start-of-measurement detector 10 from the change in conductivity of the developing layer, and the measurement time was determined on the assumption that the start-of-detection time was zero. A strip comprising a first unit and a second unit was prepared and colloidal gold with particle sizes of 20 nm and 70 nm were used as the labeling materials for the anti-hCG monoclonal antibodies as the first specific binding substances of the respective units. Further, the equal amount of the solution of the anti-hCG monoclonal antibody labeled with each of the colloidal gold, with the absorption at 520 nm made constant, was sealed into each of the space forming zones.

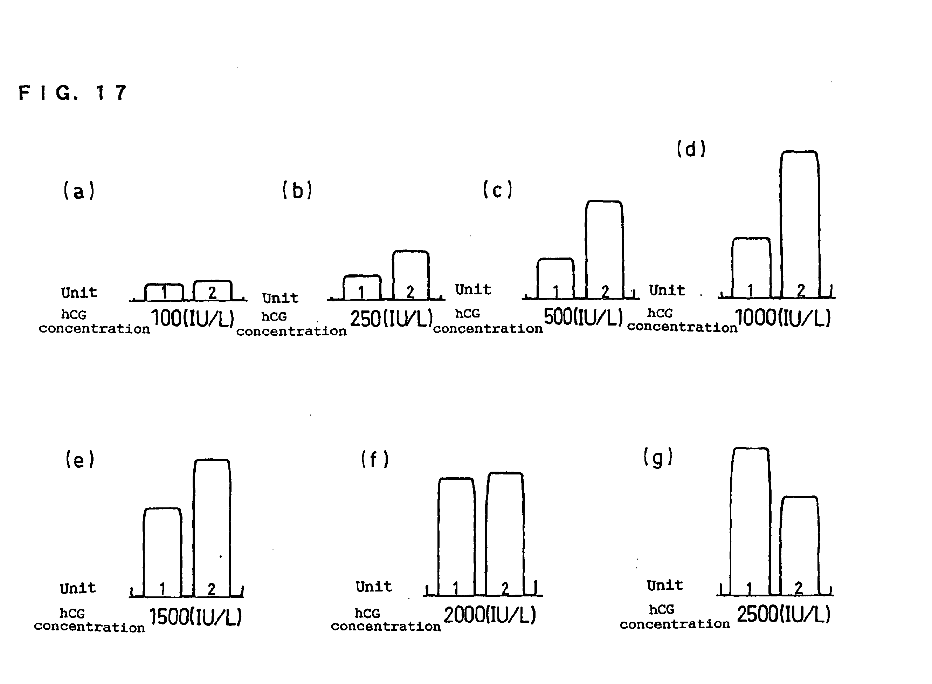

(a) to (g) in FIG. 17 represented the distribution statuses of the signal intensities in the detection zones 6 at 520 nm, measured five minutes after the detection of the sample introduction. As obvious from the drawing, since the units are disposed in parallel while providing a gap therebetween, the signal intensities in the respective units can be distinguishd when the signal intensities in a plurality of detection zones 6 are successively measured.

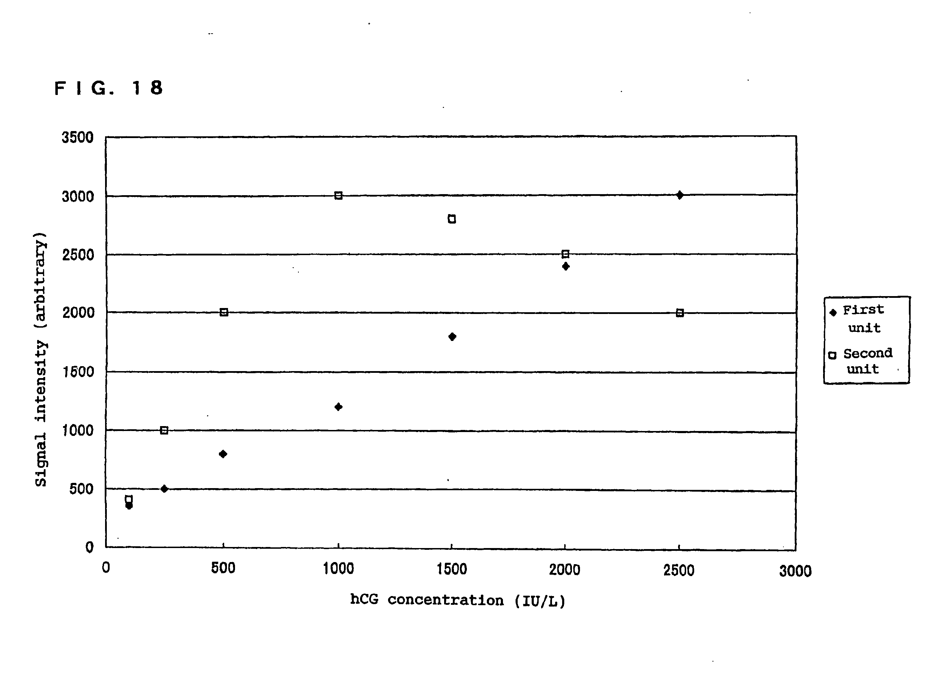

FIG. 18 is a graph showing the relationships between the hCG concentrations and the signal intensities in the detection zones 6 at 520 nm, measured five minutes after the detection of the sample introduction, when it is assumed that the height of the curve in FIG. 17 was the signal intensity in the detection zone of each of the units. The signal intensity in the second unit measured five minutes after the detection of the sample introduction increases with an increase in hCG concentration when the concentration is low (hCG concentration = 100 IU/L, 250 IU/L, 500 IU/L, 1,000 IU/L); however, on the contrary, it decreases with an increase in hCG concentration when the concentration is as high as not less than 1,500 IU/L, whereby it is found that there may exist a plurality of hCG concentrations which correspond to one signal intensity, depending on the concentration range.

In this case, that is, the presence of excess hCG in the sample causes competition between hCG not specifically bound to the first specific binding substance labeled with colloidal gold and hCG specifically bound to the first specific binding substance labeled with colloidal gold, in the specific binding reaction with the second specific binding substance in the detection zone 6, resulting in a decrease in amount of the first specific binding substance labeled with colloidal gold, which is bound in the detection zone 6, and a decrease in signal intensity, to which colloidal gold contributes, and hence the signal intensity detected in the detection zone 6 does not reflect the amount of hCG in the sample.

In the first unit, in terms of each of the hCG concentrations, the signal intensity increases dependently on the concentration, indicating that they are in the linear relationship. Although not shown in the drawing, when a sample, with the hCG concentration being substantially zero as indicated by the detector, was introduced into the strip, the signal intensity in the detection zone 6 at 520 nm was zero. This means that in the first unit, by making the particle size of colloidal gold as the labeling material smaller, the number of the anti-hCG monoclonal antibodies as the first specific binding substances which were sealed in the space forming zone increased while the amount of hCG not specifically bound to the first specific binding substance labeled with colloidal gold, which had caused the prozone phenomenon observed in the second unit, decreased, to avoid the influence of the prozone phenomenon.

It is thereby found that the hCG concentration in the sample introduced into the test strip can be calculated by previously adjusting the characteristic of the labeling material to adjust the number of the anti-hCG monoclonal antibodies as the first specific binding substances labeled with colloidal gold in the respective units and automatically adjust the composition ratio of the analyte and the specific binding substance which are capable of participating in the specific binding reaction, and by utilizing the calibration curve, from the absorption of the labeling material in the detection zone 6 in the unit with the composition ratio not influenced by the prozone phenomenon.

### Industrial Applicability

As thus described, according to the present invention, a specific binding analysis device and a specific binding analysis method can be provided, which are capable of making a sample automatically diluted without the need for an advanced device or operation, and further capable of qualitatively or quantitatively analyzing an analyte in a sample by means of a small-sized, simple measurement device in a simple, rapid and accurate manner, under little influence attributed to the concentration of the analyte in the sample.

## Claims

1. A specific binding analysis device for qualitatively or quantitatively analyzing an analyte in a sample, using a specific binding reaction between an analyte and a specific binding substance capable of specifically binding to said analyte,
said device comprising: an inlet for introducing said sample; a plurality of space forming zones for temporarily retaining said sample introduced from said inlet; a plurality of reaction fields where a specific binding reaction of said sample retained in said space forming zone occurs; and an outlet for discharging said sample from said space forming zone to said reaction field,
said specific binding reaction occurring under a different condition in each of said reaction fields.

2. The specific binding analysis device in accordance with Claim 1, wherein the number of said inlets is one.

3. The specific binding analysis device in accordance with Claim 1, wherein each of said space forming zones is different in content.

4. The specific binding analysis device in accordance with Claim 1 or 2, wherein the rate of introducing said sample from said inlet into said space forming zone is faster than the rate of discharging said sample from said outlet to said reaction field.

5. The specific binding analysis device in accordance with any of Claims 1 to 3, wherein said reaction field comprises a first specific binding substance labeled with a labeling material and a second specific binding substance, and a qualitative or quantitative analysis is performed based on a signal attributed to a specific binding reaction that said first specific binding substance and said second specific binding substance are bound to one another via said analyte.

6. The specific binding analysis device in accordance with Claim 5, wherein the characteristic of said labeling material differs in each of said reaction fields.

7. The specific binding analysis device in accordance with Claim 6, wherein the characteristic of said labeling material is a particle size or a material of said labeling material.

8. The specific binding analysis device in accordance with any of Claims 5 to 7, wherein said reaction field is a developing layer capable of developing said sample due to capillarity, a detection zone is disposed on said developing layer, and said second specific binding substance is immobilized in said detection zone.

9. The specific binding analysis device in accordance with Claim 8, wherein said first specific binding substance is retained on said developing layer.

10. The specific binding analysis device in accordance with Claim 8, wherein said sample does not move between a plurality of said developing layers.

11. The specific binding analysis device in accordance with any of Claims 1 to 10, comprising equipment for differentiating, by each of said a plurality of said reaction fields, signals attributed to specific binding sent from said reaction fields and also for successively reading out said signals.

12. A specific binding analysis method for qualitatively or quantitatively analyzing an analyte in a sample,
using a specific binding analysis device which comprises: an inlet for introducing said sample; a plurality of space forming zones for temporarily retaining said sample introduced from said inlet; a plurality of reaction fields where a specific binding reaction of said analyte in said sample retained in said space forming zone occurs; and an outlet for discharging said sample from said space forming zone to said reaction field, and is **characterized in that** said specific binding reaction occurs under a different condition in each of said reaction fields, and
adjusting at least one selected from the group consisting of a content of said space forming zone, a dilution ratio of said sample retained in said space forming zone and the characteristic of said labeling material to analyze said analyte.

13. The specific binding analysis method in accordance with Claim 12, wherein the characteristic of said labeling material is a particle size or a material of said labeling material.

14. The specific binding analysis method in accordance with Claim 12, wherein said analyte in said sample is qualitatively or quantitatively analyzed by making said space forming zone carry a solution containing a first specific binding substance, and then adjusting an amount of said solution to be carried.

15. The specific binding analysis method in accordance with Claim 12, wherein said analyte in said sample is qualitatively or quantitatively analyzed by analyzing the product of an intensity of a signal attributed to said specific binding reaction and a magnification of said dilution.

16. The specific binding analysis method in accordance with Claim 12, wherein said analyte in said sample is qualitatively or quantitatively analyzed by analyzing in said reaction fields a signal intensity of a portion where a specific binding reaction is conducted to show a signal intensity not higher than a prescribed threshold.

17. The specific binding analysis method in accordance with Claim 15, wherein said analyte in said sample is qualitatively or quantitatively analyzed by analyzing a signal intensity of a portion where a specific binding reaction is conducted to show the largest signal intensity among reaction fields where a specific binding reaction is conducted to show signal intensities not higher than said threshold.

18. The specific binding analysis method in accordance with Claim 16 or 17, wherein said threshold is less than the minimum value of a signal intensity at an intersection which is generated when overlapping corresponding curves of an analyte concentration and a signal intensity of each of said portions where a specific binding reaction is conducted, and is also less than the minimum signal intensity among the maximum values of said signal intensities of respective said corresponding curves.

19. The specific binding analysis method in accordance with any of Claims 16 to 18, wherein, when said analyte in said sample is qualitatively or quantitatively analyzed, referring to said corresponding curves of said analyte concentration and said signal intensity of said portion where a specific binding reaction is conducted to show the largest signal intensity among said reaction fields where a specific binding reaction is conducted to show signal intensities not higher than said threshold, in a case where there exist a plurality of analyte concentrations corresponding to said signal intensity of said portion where a specific binding reaction is conducted to show the largest signal intensity among said reaction fields where a specific binding reaction is conducted to show signal intensities not higher than said threshold, the smallest concentration is considered as the analyte concentration.

20. The specific binding analysis method in accordance with Claim 12, wherein said analyte in said sample is qualitatively or quantitatively analyzed by differentiating, by each of said a plurality of said reaction fields, signals attributed to specific binding sent from said reaction fields and also for successively reading out said signals.
